# EUROPEAN PATENT SPECIFICATION

(11) **EP 3 917 461 B1**
(45) Date of publication and mention of the grant of the patent: **16.07.2025**
(21) Application number: 20708985.5
(22) Date of filing: 31.01.2020
(51) Int. Cl.: A61F 2/915, A61F 2/958, A61F 2/954, A61F 2/07

(54) **EXPANDABLE LUMINAL STENTS**
EXPANDIERBARE LUMENSTENTS
ENDOPROTHÈSES LUMINALES EXPANSIBLES

(30) Priority: 01.02.2019 US 201962800078 P
(43) Date of publication of application: 08.12.2021
(73) Proprietor: Bolton Medical, Inc., Sunrise, FL 33325 (US)
(72) Inventor: ARBEFEUILLE, Samuel, Sunrise, FL 33325 (US); GARCIA, Eduardo, Alejandro, Sunrise, FL 33325 (US); LOSTETTER, Timothy, Sunrise, FL 33325 (US); MAGEN, Eitan, Sunrise, FL 33325 (US); PARANJAPE, Harshad, Sunrise, FL 33325 (US); RUSH, Scott, Lyle, Sunrise, FL 33325 (US)
(74) Representative: Graham Watt & Co LLP
(86) International application number: PCT/US2020/016218
(87) International publication number: WO 2020/160476

(56) References cited:
- EP-A1- 2 606 854
- EP-A1- 3 381 416
- WO-A1-01/35863
- WO-A1-2012/047308
- WO-A2-2007/146021
- WO-A2-2015/013344
- DE-A1- 102013 111 593
- US-A1- 2006 135 985
- US-A1- 2009 163 994
- US-A1- 2013 261 727

## Description

### BACKGROUND OF THE INVENTION

Stents are often employed to bridge a stent graft prosthesis and an arterial branch of a patient when treating aortic disease, such as aortic aneurysms. Known as "luminal stents," or "bridging stents," implantation generally includes direction of such stents in a collapsed state through a fenestration in a previously implanted stent graft. While the luminal stent can be self-expanding, balloons are often employed in combination with luminal stents that are not self-expanding in order to accommodate each patient's unique anatomy. For example, the degree of expansion required may vary along the length of the luminal stent. Also, the proper implantation may require that the force of radial expansion vary along the length of the luminal stent, such as where additional force may be required to secure a balloon expandable luminal stent at the fenestration of the previously implanted stent graft. In those cases, a first balloon and balloon catheter upon which it is mounted often must be extracted and exchanged with the second balloon catheter that has a balloon of larger diameter than its predecessor. Removal and substitution of balloons during surgery necessarily prolongs the procedure and can further traumatize tissue.

In addition, the unique anatomy of each patient typically requires customization during implantation, regardless of the design of the luminal stent, specifically in the degree of expansion of the luminal stent from a collapsed position. More specifically, the stiffness, both radially and longitudinally, may need to vary along the length of a branch stent graft, thereby requiring great precision during implantation. However, branch stent grafts generally available are limited in radial stiffness and longitudinal flexibility, and so must carefully be chosen before, or even during surgery, in order to be properly fit to the fenestration of the implanted prosthesis and accommodate the patient's anatomy. Choosing the wrong luminal stent can be problematic and even tragic in that, once deployed, stents generally cannot be removed and replaced. EP2606854 discloses a stent-graft that comprises first and second stents, which each may comprise a series of distal apices disposed distal to a proximal end of the graft, and a series of proximal apices disposed proximally beyond the proximal end of the graft. In one example, the first stent comprises a first uniform segment, and the second stent comprises a second uniform wire segment, where the first uniform segment comprises portions disposed both internal and external to the second uniform wire segment.

Therefore, a need exists for a system and method of aortic treatment that overcomes or minimizes the above mentioned problems.

### SUMMARY OF THE INVENTION

The invention generally is directed to a luminal stent assembly according to the claims. The disclosure is directed to method of implanting the luminal stent, and to a method of implanting the luminal stent assembly and the luminal stent system. The methods are not according to the invention, which is defined by the claims.

There is described, a luminal stent that includes a plurality of radially-expandable stent components, each radially-expandable stent component having a proximal end and a distal end, wherein at least one of the stent components includes struts that are joined to each other at respective opposite ends, thereby forming proximal apices and distal apices, the radially-expandable stent components being arranged in relative proximal and distal relationship to each other. A plurality of bridges link immediately proximal and distal radially-expandable stent components to each other, thereby forming the luminal stent and defining a continuous lumen, and a proximal end and a distal end of the luminal stent, wherein the axial stiffness of the luminal stent decreases from the proximal end to the distal end of the luminal stent as a consequence of a decrease in the number of bridges spanning, also referred to as "linking," the radially-expandable stent components with increasing distance from the proximal end of the luminal stent, and the radial stiffness of the luminal stent decreases from the proximal end to the distal end of the luminal stent as a consequence of at least one of a increase in the length of the struts of the radially- expandable stent components and a decrease in thickness of the stents of the radially- expandable stent components with increased distance from the proximal end of the luminal stent.

There is also described, a luminal stent assembly that includes a luminal stent and at least one of the luminal graft component and a polymeric coating at the luminal stent. The luminal stent includes a plurality of radially-expandable stent components, each radially- expandable stent component having a proximal end and a distal end, at least one of the stents including struts, wherein the struts include opposite ends and are joined to each other at the respective opposite ends, thereby forming proximal apices and distal apices, the radially expandable stent components being arranged in relative proximal and distal relationship to each other. A plurality of bridges of the luminal stent link immediately proximal and distal radially-expandable stent components to each other, thereby forming the luminal stent and defining a continuous lumen at a proximal and a distal end of the luminal stent, wherein the axial stiffness of the luminal stent decreases from the proximal end to the distal end of the luminal stent as a consequence of a decrease in the number of bridges spanning the radially-expandable stent components with increasing distance from the proximal end of the luminal stent, and the radial stiffness of the luminal stent decreases from the proximal end to the distal end of the luminal stent as a consequence of at least one of a increase in the length of the struts of the radially-expandable stent components and a decrease in thickness of the stents of the radially-expandable stent components with increased distance from the proximal end of the luminal stent. The luminal stent assembly further includes at least one of a luminal graft component and a polymeric coating at the luminal stent, to thereby form the luminal stent assembly.

There is further described, a luminal stent assembly that includes a luminal stent and a balloon within the luminal stent when the luminal stent is in a collapsed position and having a greater diameter at one end and at an opposite end when inflated. The luminal stent includes a plurality of radially-expandable stent components, each radially-expandable stent component having a proximal end and a distal end, at least one of the stent components including struts, the struts including opposite ends that are joined to each other at the respective opposite ends, thereby forming proximal apices and distal apices. The radially-expandable stent components are arranged in relative proximal and distal relationship to each other, and have a plurality of bridges linking immediately proximal and distal radially-expandable stent components to each other, wherein the axial stiffness of the luminal stent decreases from the proximal end to the distal end of the luminal stent as a consequence of a decrease in the number of bridges spanning the radially-expandable stent components with increasing distance from the proximal end of the luminal stent, and the radial stiffness of the luminal stent decreases from the proximal end to the distal end of the luminal stent as a consequence of at least one of a increase in the length of the struts of the radially-expandable stent components and a decrease in thickness of the stents of the radially-expandable stent components with increased distance from the proximal end of the luminal stent. A balloon is within the luminal stent when the luminal stent is in a collapsed state and has a greater diameter at one end than an opposite end when inflated.

There is further described, a luminal stent assembly that includes a luminal stent, at least one of a luminal graft component and a polymeric coating at the luminal stent, to thereby form the luminal stent assembly, the luminal stent assembly having a proximal end and a distal end, and a balloon within the luminal stent when the luminal stent is in a collapsed position and having a greater diameter at one end and at an opposite end when inflated. The luminal stent includes a plurality of radially-expandable stent components, each radially-expandable stent component having a proximal end and a distal end, at least one of the stent components including struts, the struts including opposite ends that are joined to each other at the respective opposite ends, thereby forming proximal apices and distal apices. The radially-expandable stent components are arranged in relative proximal and distal relationship to each other, and have a plurality of bridges linking immediately proximal and distal radially-expandable stent components to each other, wherein the axial stiffness of the luminal stent decreases from the proximal end to the distal end of the luminal stent as a consequence of a decrease in the number of bridges spanning the radially-expandable stent components with increasing distance from the proximal end of the luminal stent, and the radial stiffness of the luminal stent decreases from the proximal end to the distal end of the luminal stent as a consequence of at least one of a increase in the length of the struts of the radially-expandable stent components and a decrease in thickness of the stents of the radially-expandable stent components with increased distance from the proximal end of the luminal stent. A balloon is within the luminal stent when the luminal stent is in a collapsed state and has a greater diameter at one end than an opposite end when inflated.

There is further described, a stent graft system that includes a luminal stent assembly that includes a luminal stent having a plurality of radially-expandable stent components radially-expandable stent component having approximately an end and a distal end, at least one of the stent components including struts, where the struts include opposite ends and are joined to each other at the respective opposite ends, thereby forming proximal apices and distal apices, the radially-expandable stent components being arranged in relative proximal and distal relationship to each other, and a plurality of bridges link immediately proximal and distal radially-expandable stent components to each other, wherein the axial stiffness of the luminal stent decreases from the proximal end to the distal end of the luminal stent as a consequence of a decrease in the number of bridges spanning the radially-expandable stent components with increasing distance from the proximal end of the luminal stent, and the radial stiffness of the luminal stent decreases from the proximal end to the distal end of the luminal stent as a consequence of at least one of a increase in the length of the struts of the radially-expandable stent components and a decrease in thickness of the stents of the radially-expandable stent components with increased distance from the proximal end of the luminal stent. A plurality of bridges linking immediately proximal and distal radially-expandable stent components to each other. At least one of the luminal graft component and the polymeric coating is at the luminal stent. A balloon is within the luminal stent when a luminal stent is in a collapsed position and has a greater diameter at one end and an opposite end when inflated.

There is further described, a luminal stent assembly that includes a luminal stent and at least one of a luminal graft component and a polymeric coating. The luminal stent includes a plurality of radially-expandable stent components, each radially-expandable stent component having a proximal end and a distal end, at least one of the stent components including struts, wherein the struts include opposite ends and are joined to each other at the respective opposite ends, thereby forming proximal apices and distal apices, the radially-expandable stent components being nested in relative proximal and distal relationship to each other. A plurality of bridges link immediately proximal and distal radially-expandable stent components to each other at at least one of the respective proximal apices and respective distal apices, thereby forming the luminal stent and defining a continuous lumen, a proximal end, and a distal end of the luminal stent. At least one of a luminal graft component and a polymeric coating is at the luminal stent to thereby form the luminal stent assembly, wherein the luminal stent assembly has a proximal end and a distal end.

There is further described, a luminal stent assembly that includes at least one luminal stent, at least one of a luminal graft component and a polymeric coating, and at least one stent distal to the luminal stent. The luminal stent includes a plurality of radially-expandable stent components, each radially-expandable stent component having a proximal end and a distal end, at least one of the stent components including struts, wherein the struts include opposite ends and are joined to each other at the respective opposite ends, thereby forming proximal apices and distal apices, the radially-expandable stent components being in relative proximal and distal relationship to each other. A plurality of bridges link, immediately proximal and distal radially-expandable stent components to each other, thereby forming the luminal stent and defining a continuous lumen, a proximal end, and a distal end of the luminal stent. At least one of the luminal graft component and the polymeric coating are at the luminal stent. At least one stent is distal to the luminal stent and is linked to the luminal stent by at least one of the luminal graft component and the polymeric coating to thereby form the luminal stent assembly, the luminal stent assembly having a proximal end at the luminal stent and a distal end at the stent distal to the luminal stent. The axial stiffness of the luminal stent assembly decreases from the proximal end to the distal end of the luminal stent assembly as a consequence of a space between the luminal stent and the at least one stent distal to the luminal stent, and the radial stiffness of the luminal stent is greater than the radial stiffness of the at least one distal stent.

There is also described, a plurality of radially-expandable stent components. Each radially-expandable stent component has a proximal end and a distal end, at least one of the stent components including struts, wherein the struts include opposite ends and are joined to each other at the respective opposite ends, thereby forming proximal apices and distal apices, the radially-expandable stent components being in relative proximal and distal relationship to each other. The radial stiffness of each radially expandable stent component is less than that of each radially expandable stent component proximal to it and greater than that of each radially-expandable stent component distal to it. At least one of a luminal graft component and a polymeric coating link the plurality of radially-expandable stent components.

There is also described, a stent graft assembly that includes a luminal stent assembly that includes a luminal stent having a plurality of radially-expandable stent components, at least one of the stent components including struts, where the struts include opposite ends and are joined to each other at the respective opposite ends, thereby forming proximal apices and distal apices, the radially-expandable stent components being arranged in relative proximal and distal relationship to each other, and a plurality of bridges link immediately proximal and distal radially-expandable stent components to each other, wherein the axial stiffness of the luminal stent decreases from the proximal end to the distal end of the luminal stent as a consequence of a decrease in the number of bridges spanning the radially-expandable stent components with increasing distance from the proximal end of the luminal stent, and the radial stiffness of the luminal stent decreases from the proximal end to the distal end of the luminal stent as a consequence of at least one of a increase in the length of the struts of the radially-expandable stent components and a decrease in thickness of the stents of the radially-expandable stent components with increased distance from the proximal end of the luminal stent. A plurality of bridges linking immediately proximal and distal radially-expandable stent components to each other. At least one of the luminal graft component and the polymeric coating is at the luminal stent. A balloon is within the luminal stent when a luminal stent is in a collapsed position. A fenestrated stent graft of the luminal stent system defines at least one fenestration, wherein the luminal stent has a diameter less than the fenestration when in a collapsed position, and is expandable to a diameter that fixes the proximal end of the luminal stent within the fenestration, whereby the distal end of the luminal stent extends radially from stent graft.

There is also described, a method of implanting a stent graft system that includes delivering a fenestrated stent graft of the stent graft system to a branched artery of a subject, wherein a fenestration defined by the fenestrated stent graft lies with a proximal end of the branch artery. A luminal stent of the luminal stent system has a plurality of radially-expandable stent components, at least one of the stent components including struts, where the struts include opposite ends and are joined to each other at the respective opposite ends, thereby forming proximal apices and distal apices, the radially-expandable stent components being arranged in relative proximal and distal relationship to each other, and a plurality of bridges link immediately proximal and distal radially-expandable stent components to each other, wherein the axial stiffness of the luminal stent decreases from the proximal end to the distal end of the luminal stent as a consequence of a decrease in the number of bridges spanning the radially-expandable stent components with increasing distance from the proximal end of the luminal stent, and the radial stiffness of the luminal stent decreases from the proximal end to the distal end of the luminal stent as a consequence of at least one of a increase in the length of the struts of the radially-expandable stent components and a decrease in thickness of the stents of the radially-expandable stent components with increased distance from the proximal end of the luminal stent. The luminal stent of the stent graft system is delivered at least partially through the fenestration and into the arterial branch, the luminal stent having a proximal end at the fenestration of the fenestrated stent graft and a distal end extending radially outward from the fenestrated stent graft. A luminal stent is radially expanded within the fenestration and the arterial branch by inflating a balloon within the luminal stent that has a greater diameter at the proximal end of the luminal stent and at the distal end of the luminal stent, thereby implanting the stent graft system.

There is also described, a method of implanting a stent graft system that includes delivering a fenestrated stent graft of the stent graft system to a branch artery of a subject, wherein the fenestration defined by the fenestrated stent graft aligns with the proximal end of the branch artery. A luminal stent assembly of a stent graft system is delivered at least partially through the fenestration and into the arterial branch, the luminal stent assembly thereby bridging the fenestrated stent graft and the arterial branch. The luminal stent assembly includes a plurality of stents aligned longitudinally and connected by bridges to form a luminal stent, wherein the axial stiffness of the luminal stent decreases from the proximal end to the distal end of the luminal stent as a consequence of a decrease in the number of bridges spanning the radially-expandable stent components with increasing distance from the proximal end of the luminal stent, and the radial stiffness of the luminal stent decreases from the proximal end to the distal end of the luminal stent as a consequence of at least one of a increase in the length of the struts of the radially-expandable stent components and a decrease in thickness of the stents of the radially-expandable stent components with increased distance from the proximal end of the luminal stent. At least one stent distal to the luminal stent has a radial stiffness less than that of the luminal stent, and at least one of a luminal graft component and a polymeric coating linking the luminal stent and the stent distal to the luminal stent. The luminal stent assembly is radially expanded within the fenestration and the arterial branch by inflating the balloon within the luminal stent assembly, wherein the balloon has a diameter at the luminal stent greater than at the stent distal to the luminal stent when inflated, thereby implanting the stent graft system.

This invention has several advantages. For example, by varying the radial and axial stiffness of a luminal, or bridging, stent, the physician can place a proximal portion of the luminal stent that is radially stiff, relative to a distal end of the luminal stent, within a fenestrated opening of a fenestrated stent graft prosthesis, thereby allowing for a better seal at the stent graft prosthesis, and reducing potential for the luminal stent to dislodge from the fenestration by, at least on one embodiment, forming an hour-glass configuration on either side of the fenestration. Simultaneously, the distal portion of the luminal stents, where radial stiffness is low relative to the proximal portion of the luminal stent, can be placed inside of a targeted vessel, thereby maintaining appropriate radial support, consequently reducing the potential of the vessel to be occluded by thrombus formation, while allowing for axial flexibility within the vessel.

Optional inclusion of a balloon having a greater diameter at the proximal end of the luminal stent during implantation minimizes or eliminates the need to remove a first balloon and substitute it with a second balloon to preferentially flare a proximal end of the luminal stent. This reduces the time requirement of the overall procedure and reduces trauma to the patient. Marker band locations and configurations can be employed to show the proximal and distal ends of the stent along with location of a transitional area of the balloon having greater and lesser expanded diameters, thereby aiding the physician and placement of the luminal stent in the fenestration, and ensuring that a flared area of the stent is properly engaged with the fenestration opening in the fenestrated stent graft. Also optionally, the distal portion of the luminal stent of the invention can be left uncovered, thereby enabling the luminal stent to be employed in bifurcated vessels without obstructing blood flow to either the branch vessel or the bifurcation, as opposed to current procedures where the physician employs a covered stent and then deploys an uncovered self-expanding stent into the area of the bifurcation.

### BRIEF DESCRIPTION OF THE DRAWINGS

FIG. I is a side (laser-cut flat pattern) view of one version of a luminal stent that decreases in axial stiffness with increasing distance from a proximal end.
FIG. 1A is a detail of FIG. 1 showing junctures of struts of radially-expandable stent components of the luminal stent that define proximal and distal apices.
FIG. IB is a cross-section of FIG. 1 taken along line 1B-1B showing a lumen defined by a luminal stent.
FIG. 1C is a detail of FIG. 1 showing a bridge between distal and proximal apices of respective proximal and distal radially-expandable stents of the luminal stent.
FIG. ID is a side view of the version of the luminal stent shown in FIG. 1, demonstrating axial flexibility of the luminal stent.
FIG. 2 is a side view of the luminal stent of FIG. 1 in a collapsed position.
FIG. 3 is a side view of the luminal stent of FIG. 1 in a collapsed position and within which is a collapsed balloon.
FIG. 4 is a side view of the luminal stent of FIG. 1 after inflation of the balloon.
FIG. 5 is a side view of another version of a luminal stent, wherein radial stiffness decreases with increasing distance from a proximal end of the luminal stent as a consequence of increasing strut length of radially- expandable stent components of the luminal stent with increasing distance from the proximal end of the luminal stent.
FIG. 6 is a side view of the luminal stent of FIG. 5 when in a collapsed position.
FIG. 7 is a side view of a stent that is made up of struts that meet at opposite ends and define proximal and distal apices.
FIG. 7 A is an end view taken along line 7A-7A, showing cross-sections of struts of the stent of FIG. 7.
FIG. 7B is a detail of FIG. 7A, showing a width W, indicating "thickness" of struts as that term is employed herein, and a depth D distinct from "thickness" of struts.
FIG. 8 is a side view of another version of a luminal stent, wherein the decrease in axial stiffness with increasing distance from the proximal end of the luminal stent is stepped at junctures.
FIG. 9 is a side view of yet another version of a luminal stent, wherein the decreasing radial stiffness with increasing distance from the proximal end of the luminal stent is stepped.
FIG. 10 is a side view of one version of a luminal stent assembly wherein a luminal stent is covered on an inside surface with a luminal graft component.
FIG. 10A is a cross-section of the luminal stent assembly of FIG.10 taken along line 10A-10A, showing that the luminal graft component is within the luminal stent component of the luminal stent assembly.
FIG. 11 is a side view of another version of a luminal stent assembly, wherein a most-proximal radially-expandable stent component extends beyond a distal end of a luminal graft component of the luminal stent assembly.
FIG. 12 is a side view of still another luminal stent assembly, wherein a luminal stent component exhibits decreasing axial stiffness with increasing distance from proximal end and a luminal graft component extends distally from the luminal stent component, and links the luminal stent component to a plurality of distal radially-expanding stents.
FIG. 13 is a side view of yet another luminal stent assembly, wherein a luminal stent component exhibits decreasing radial stiffness with increasing distance from a proximal end and luminal graft component extends distally from the luminal stent component, and links the luminal stent component to a plurality of distal radially-expanding stents.
FIG. 14 is a side view of another luminal stent assembly, wherein distal stents include pairs of radially-expanding stent components and struts of the distal stents are longer than struts of the luminal stent component.
FIG. 15 is a side view of another version of the luminal stent assembly, wherein a polymeric coating covers a luminal stent of the luminal stent assembly, wherein the luminal stent exhibits decreasing axial stiffness with increasing distance from a proximal end of the luminal stent assembly.
FIG. 15A is a cross-section of the luminal stent assembly of FIG. 15, taken along line 15A-15A, showing that, in this version, the polymeric coating covers both the inside surface and the outside surface of the luminal stent component.
FIG. 16 is a side view of another version of a luminal stent assembly, a polymeric coating links a luminal stent with distal stents.
FIG. 17 is a side view of still another version of a luminal stent assembly, wherein the luminal stent has shorter struts than struts of distal stents to which it is linked by a polymeric coating.
FIG. 18 is a side view of a balloon suitable for use in an version of the luminal stent assembly, wherein the balloon when inflated has a greater diameter at a proximal end than at a distal end of the balloon.
FIG. 19 is a side view of another version of the luminal stent assembly, wherein the balloon shown in FIG. 18 is inflated inside a luminal stent and distal stents of the luminal stent assembly.
FIG. 19A is a detail of the luminal stent of FIG. 19.
FIG. 19B is a side view of the luminal stent assembly of FIG. 19 after radial expansion within a fenestration of a fenestrated stent graft.
FIG. 20 is a side view of still another version of a luminal stent assembly, wherein a luminal stent exhibits decreasing axial stiffness with increasing stiffness from a proximal end of the luminal stent assembly, and a polymeric coating links luminal stent with distal stents of the luminal stent assembly.
FIG. 20A is a cross-section of the luminal stent assembly of FIG. 20 taken along line 20A-20A.
FIG. 21, is another embodiment of a luminal stent of the invention, wherein radially-expanding stent components of a luminal stent are nested, and bridged at proximal apices and distal apices, and the axial stiffness of the luminal stent decreases with increasing distance from a proximal end of the luminal stent.
FIG. 22 is another embodiment of a luminal stent of the invention, wherein radially-expanding stent components of a luminal stent are nested and bridged at only proximal apices, and wherein the luminal stent exhibits decreasing axial stiffness with increasing distance from a proximal end of the luminal stent assembly.
FIG. 23 is still yet another embodiment of a luminal stent of the invention, wherein radially-expanding stent components of a luminal stent are nested and bridged at only the distal apices, and wherein the luminal stent exhibits decreasing axial stiffness with increasing distance from a proximal end of the luminal stent assembly.
FIG. 24 is another embodiment of a luminal stent assembly of the invention, wherein a portion of radially-expanding stent components of a luminal stent are not nested and another portion of the radially-expanding stent components are nested, and further including distal stents that are nested and are linked to each other and to the luminal stent by a polymeric coating, the distal stents exhibiting least one of decreased axial stiffness and radial stiffness relative to the luminal stent of the luminal stent assembly.
FIG. 25 is a side view of another version of a luminal stent assembly, wherein a plurality of stents that are not nested collectively exhibit decreasing radial stiffness with increasing distance from a proximal end of the luminal stent assembly, and wherein the radially-expanding stents are linked by a polymeric coating.
FIG. 26 is a side view of a version another luminal stent assembly, wherein a plurality of stents that are nested collectively exhibit decreasing radial stiffness with increasing distance from a proximal end of the luminal stent assembly, and wherein the radially-expanding stents are linked by a polymeric coating.
FIG. 27 is yet another version of another luminal stent assembly, including a luminal bridging stent of the invention and a fenestrated stent graft, wherein a balloon in a collapsed position is within the luminal bridging stent, and a luminal stent of the luminal stent assembly has been implanted within a fenestration of an fenestrated stent graft that has been implanted within a subject.
FIG. 28 is a side view of the luminal stent assembly shown in FIG. 27 following inflation of the balloon, thereby securing the luminal stent of the luminal stent assembly within the fenestration of the fenestrated stent graft.
FIG. 29 is an exploded side view of one version of a stent graft delivery system.
FIG. 30A is a side view of the stent graft delivery system shown in FIG. 29, but in assembled form and, wherein the introducer sheath, containing a stent graft of the stent graft delivery system, has been delivered to an arterial aneurysm of a patient.
FIG. 30B is a side view of the stent graft delivery system of FIG. 30A, following proximal retraction of the introducer sheath along the stent graft delivery device, to thereby expose the stent graft, which is held in a radially constricted position by a wire of the stent graft delivery system.
FIG. 30C is a side view of the stent graft delivery system shown in FIGs. 30A and 10B, following partial retraction of the wire from ligatures that, when linked by the wire, holds the stent graft in a partially radially constricted position, while the remainder of the stent graft is in a radially expanded position.
FIG. 30D is a side view of the graft prosthesis delivery system shown in FIGs. 30A-30C, following full retraction of the wire from the stent graft, whereby the stent graft is in a radially expanded position along its entire length.
FIG. 30E is a side view of the stent graft delivery system shown in FIGs. 30A through 30D, following retraction of the remainder of the stent graft delivery system not implanted at the aneurysm, whereby implantation of the stent graft at the aneurysm of the patient is complete.
FIG. 31 is a detail of the branch luminal stent assembly of FIG. 30E following inflation of a balloon within the luminal stent assembly.

### DETAILED DESCRIPTION OF THE INVENTION

The features and other details of the invention, either as steps of the invention or as combinations of parts of the invention, will now be more particularly described and pointed out in the claims. It will be understood that the particular embodiments of the invention are shown by way of illustration and not as limitations of the invention. The principal features of this invention can be employed in various embodiments without departing from the scope of the invention.

The present invention generally is directed to a luminal stent assembly as shown in the claims. The invention is useful in methods of implanting the luminal stent, the stent graft assembly and the luminal stent system in a branched artery to treat diseased tissue at the branched artery. The luminal stent includes a plurality of radially-expandable stent components and a plurality of bridges linking immediately proximal and distal radially-expandable stent components to each other. In one embodiment, the axial stiffness of the luminal stent decreases from the proximal end to the distal end of the luminal stent as a consequence of a decrease in the number of bridges spanning, also referred to as "linking," the radially-expandable stent components with increasing distance from the proximal end of the luminal stent, and the radial stiffness of the luminal stent decreases from the proximal end to the distal end of the luminal stent as a consequence of at least one of a increase in the length of the struts of the radially-expandable stent components and a decrease in thickness of the stents of the radially-expandable stent components with increased distance from the proximal end of the luminal stent.

The luminal stent assembly, includes the luminal stent assembly of the invention and at least one of a luminal graft component and a polymeric coating at the luminal stent. The luminal stent system includes a luminal stent assembly from the invention, at least one of the luminal graft component and a polymeric coating at the luminal stent, a balloon within the luminal stent when the luminal stent is in a collapsed position that has a greater diameter at one end than an opposite end when inflated, and a fenestrated stent graft defining at least one fenestration, wherein the luminal stent has a diameter of less than the fenestration when in a collapsed position, and is expandable to a diameter that fixes the proximal end of the luminal stent within the fenestration, whereby the distal end of the luminal stent extends radially from the stent graft.

One version of the method using the invention includes delivering the fenestrated stent graft of the luminal stent system to a branched artery, delivering a luminal stent of the stent graph system at least partially through a fenestration of the fenestrated stent graft, and radially expanding the proximal end of the luminal stent within the fenestration, and expanding the distal end of the luminal stent within an arterial branch of a patient by inflating a balloon within the luminal stent. In another version the method includes delivering a luminal stent assembly that includes a plurality of stents aligned longitudinally and connected by bridges to form a luminal stent, the stent distal to the luminal stent having a radial stiffness less than that of the radial stent, and at least one of the luminal graft component and a polymeric coating linking the luminal stent and the stent distal to the luminal stent. The luminal stent is radially-expanded within the fenestration of a fenestrated graft and within an arterial branch of the patient by inflating the balloon within the luminal stent assembly and when the balloon has a greater diameter at the luminal stent than at the distal to the luminal stent when inflated, thereby implanting the stent graph system.

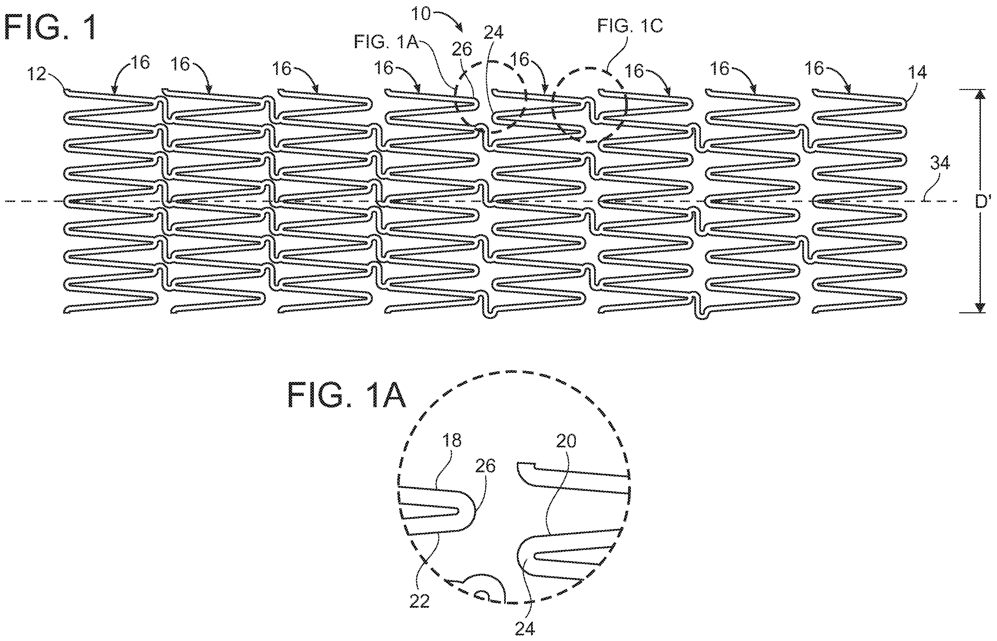

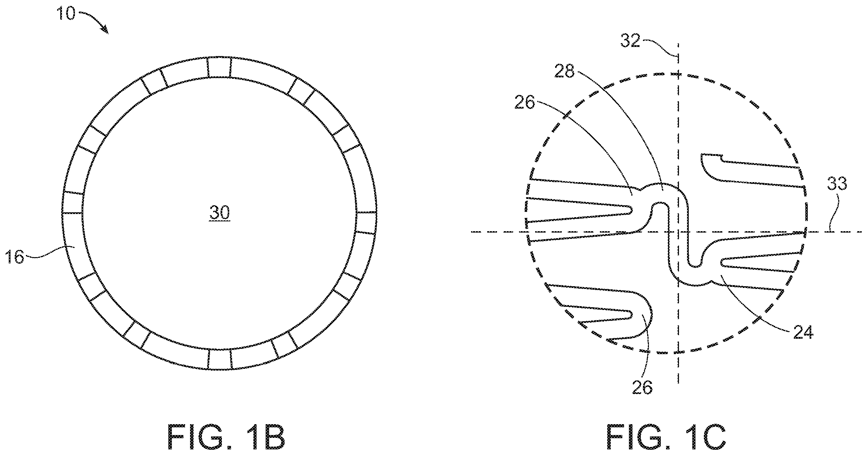

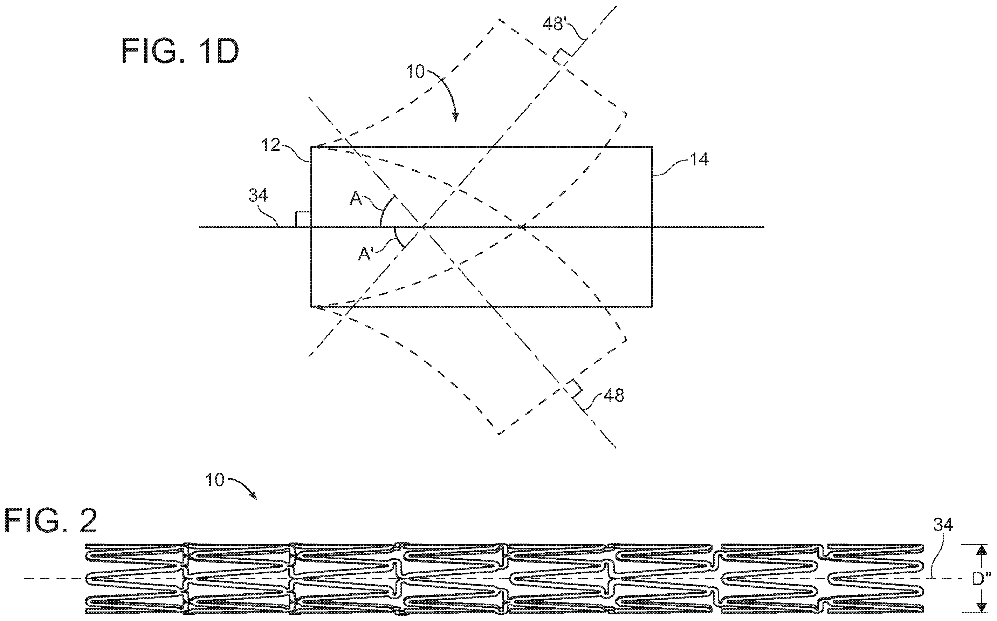

FIG. 1 is representative of a luminal stent. Luminal stent 10 includes proximal end 12 and distal end 14 and is made up of radially-expandable stent components 16. Struts 18 of each radially-expandable stent component 16 include proximal end 20 and distal end 22 opposite to each other. Respective proximal ends 20 of struts 16 are joined, as are distal ends 22 of struts 16, thereby forming proximal apices 24 and distal apices 26, as shown in FIG. 1 A. Radially-expandable stent components 16 are arranged in relative proximal and distal relationship to each other, extending from proximal end 12 of luminal stent 10 to distal end 14 of luminal stent 10.

Referring back to FIG. 1, radially-expandable stent components 16 are linked by bridges 28 between immediately proximal and distal radially-expandable stent components 16 to each other to thereby form luminal stent 10 and define continuous lumen 30 extending from proximal end 12 to distal end 14 of luminal stent 10, as shown in FIG. IB, which is across-section taken along line IB-IB of FIG. 1.

In the embodiment shown in FIG. 1, at least a portion of bridges 28 link at least a portion of distal apices 26 and proximal apices 24 of respective proximal and distal radially-expandable stent components 16. It is to be understood, however, that in other versions, not shown, bridges 28 can link immediately distal and proximal radially-expandable stent components 16 at struts 18 of respective radially-expandable stent components 16 or between distal apex 26 or proximal apex 24 of a radially-expandable stent component 16 and a strut 18 of the respective immediately-distal or immediately proximal radially-expandable stent component 16. In another version, also not shown, apices of one radially-expandable stent component can be nested between proximal and distal apices of respective distal and proximal radially-expandable stent components, and can be joined by bridges between struts of immediately proximal and distal radially-expandable stent components, or between apices and struts of those components.

In an version, such as that shown in FIG. 1C, at least a portion of bridges 28 have a longitudinal axis 32 transverse to longitudinal axis 33. Longitudinal axis 33 is parallel to longitudinal axis 34 of luminal stent 10 of continuous lumen 30 defined by radially-expandable stent components 16 of luminal stent 10. As a consequence of this orientation of bridges 28, luminal stent 10 is axially flexible.

As can be seen in FIG. ID, axis 34 normal to a proximal end 12 of luminal stent 10 can have variable angle A', A" with axes 48', 48", respectively, normal to distal end 14 of luminal stent 10 by flexation of luminal stent 10, as shown in FIG. ID. This flexation is referenced herein as "axial flexibility," or "bending flexibility." "Axial stiffness," or "bending stiffness," as defined herein, means resistance to axial flexation.

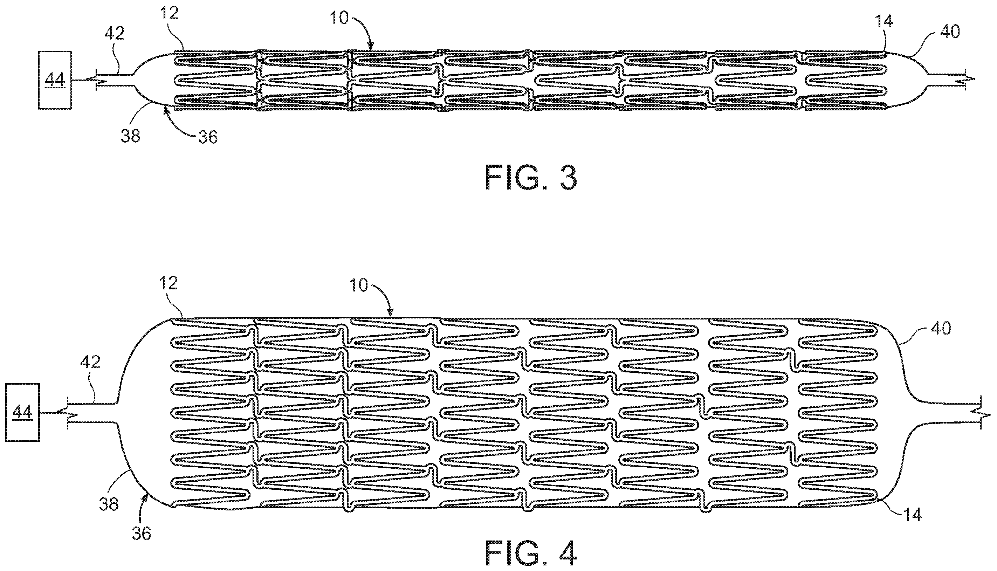

Luminal stent 10, as shown in the embodiment of FIG. 1, is in a radially-expanded position and has diameter D'. In FIG. 2, luminal stent 10 is shown in a collapsed position, where diameter D" of luminal stent 10 is less than that of diameter D' of luminal stent 10 when in a radially collapsed position, as shown in FIG. 1. In one version, radially-expandable stent components 16 exhibit resistance to radial expansion from the radially collapsed position, shown in FIG. 2, to the radially- expanded position, shown in FIG. 1. In this version, radial expansion of radially-expandable stent components 16 is effected by balloon 36 disposed within luminal stent 10 when the luminal stent 10 is in a collapsed position, as shown in FIG. 3. Balloon 36 includes proximal end 38 located at proximal end 12 of luminal stent 10 and distal end 40 located at distal end 14 of luminal stent 10. Balloon catheter 42 extends from distal end 40 of balloon 36 and is employed to inflate balloon 36 by directing fluid from a suitable source 44 to balloon during implantation of luminal stent 10. Inflation of balloon 36 by direction of fluid from fluid source 44 through balloon catheter 42 causes radial expansion of luminal stent 10 to an expanded position, as shown in FIG. 4. Radially-expandable stent components 16 are fabricated of a suitable material, such as at least one member selected from the group consisting of stainless steel, cobalt (Co), Nitinol (Ni-Ti), cobalt-chromium alloy (605L), and titanium (Ti).

In another version, radial stiffness of radially-expandable stent components 16 includes resistance to radial collapse from a radially expanded position. In this version, at least a portion of radially-expandable stent components 16 of luminal stent 10 include at least one member selected from the group consisting of stainless steel, cobalt (Co), Nitinol (Ni-Ti), cobalt-chromium alloy (605L), and titanium (Ti). In still another version, radially-expandable stent components 16 include a shape-memory elastic metal, such as Nitinol. In a specific version the shape-memory elastic metal of the radially-expandable stent components 16 includes Nitinol. In another specific version the shape-memory elastic metal of the radially-expandable stent components 16 is Nitinol. In still another version, a portion of radially-expandable stent components 16 of luminal stent 10 include, or are formed of a shape-memory elastic metal, such as Nitinol, and are self-expanding, and another portion of radially-expandable stent components 16 are balloon-expandable, and are formed of, for example, at least one member selected from the group consisting of stainless steel, cobalt (Co), Nitinol (Ni-Ti), cobalt-chromium alloy (605L), and titanium (Ti).

"Radial-expandability," as defined herein, means an ability to increase in a dimension normal to a longitudinal axis of an elongate object, such as luminal stent, 10 when inflated from a collapsed position, shown in FIG. 3, to an inflated position, shown in FIG. 4. Self expanding stents would not need a balloon to expand unless they were secured to the balloon.

"Radial-contractability," as defined herein, means the opposite of radial expandability.

"Radial stiffness," as defined herein, means resistance to at least one of radial-expansion and radial-contraction of a diameter of a radially-expandable stent component.

A luminal stent 10 has an axial stiffness that decreases from proximal end 12 to distal end 14 of luminal stent 10. The mechanism of reduced axial stiffness along a longitudinal length of luminal stent 10 from proximal end 12 to distal end 14 can be a consequence of any suitable mechanism known in the art. In one embodiment, shown in FIG. 1, decreasing axial stiffness from proximal end 12 to distal end 14 of luminal stent 10 is a consequence of a diminishment of the number of bridges 28 spanning radially-expandable stent components 16 with increased distance from proximal end of luminal stent 10.

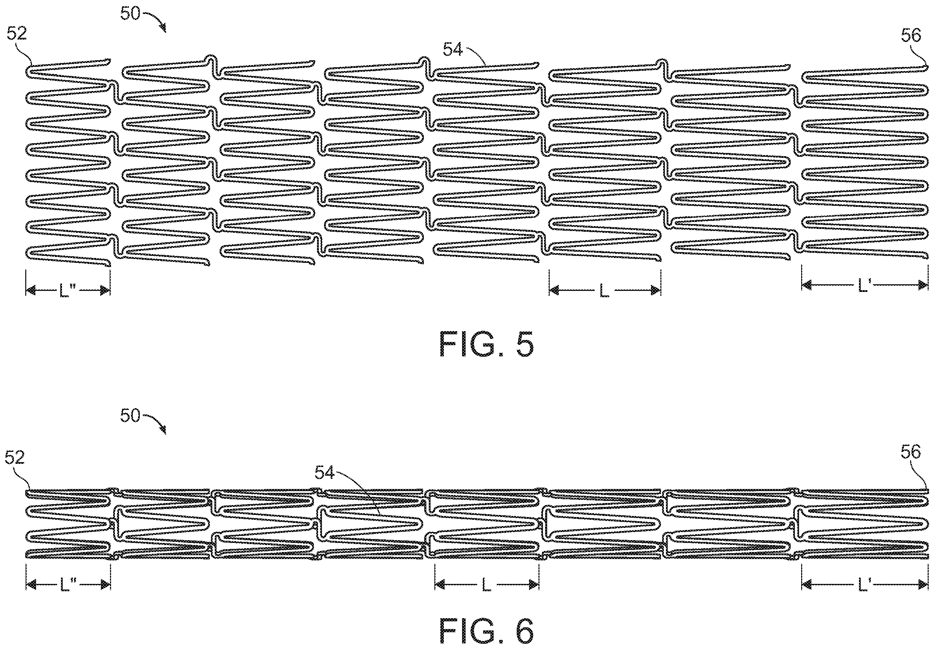

In another version, radial stiffness of luminal stent 50 decreases with increasing distance from proximal end 52 of luminal stent 50 as a consequence of any suitable mechanism known in the art. For example, as shown in FIG. 5, wherein luminal stent 50 is shown in an expanded position, an increase in length L of struts of radially-expandable stent components 16 with increased distance from proximal end 52 of luminal stent 50 toward distal end 56 causes a decrease in radial stiffness with increasing distance from proximal end 52. Specifically, L' is greater than L", and can be progressively so with increasing distance from proximal end 52 toward distal end 56 of luminal stent 50. FIG. 6 is a side view of luminal stent 50 in a collapsed position.

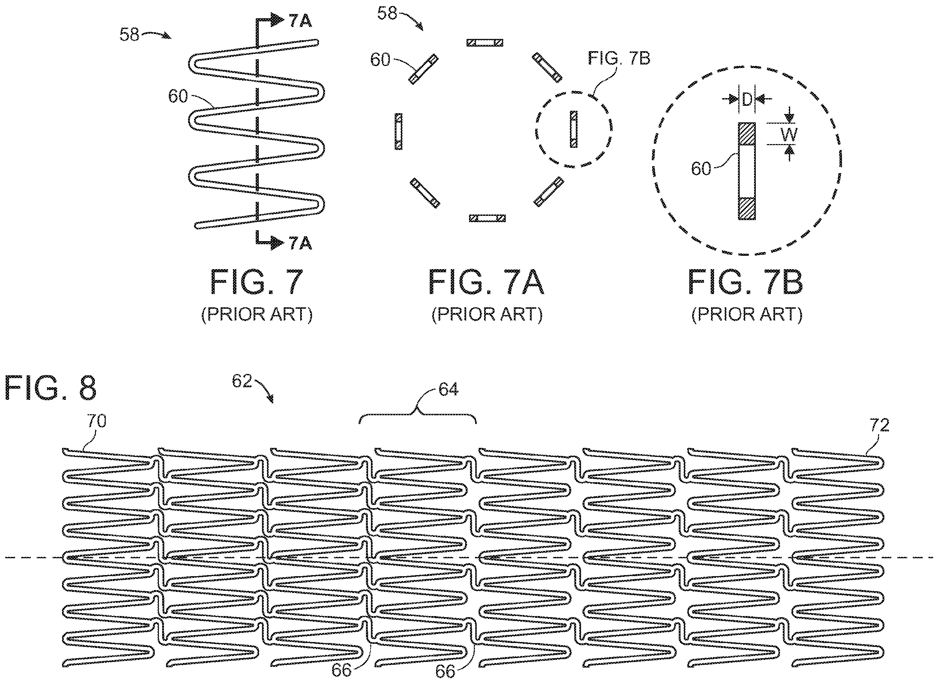

In yet another version, not shown, decreasing radial stiffness with increasing distance from a proximal end of a stent component is a consequence of a decrease in thickness of at least a portion of struts of radially expandable stent components with increased distance from proximal end of luminal stent. For example, FIG. 7 is a radially expandable stent 58. FIG. 7A is a cross section of radially-expandable stent 58, shown in FIG 7, taken along line 7A-7A. FIG. 7B is a detail of a cross section of one strut 60 of radially-expandable stent 58. "Thickness" of struts, as referenced herein, whether reference is made to a radially-expandable stent component of a luminal stent or of a radially-expandable stent, means the width W of the strut in a plane tangential to a lumen defined by the luminal stent, as shown in FIG. 7, as opposed to a depth D, also shown in FIG. 7.

In other versions, not shown, decreasing radial stiffness with increasing distance from a proximal end of a luminal stent is a consequence of both an increase in the length of the struts of the radially-expandable stent components and a decrease in thickness of the struts of the radially expandable stent components with distance from the proximal end of the luminal stent. In still another version, also not shown, a luminal stent exhibits both a decrease in axial stiffness as a consequence of a decrease in the number of bridges spanning the radially-expandable stent components with increasing distance from a proximal end of a luminal stent, and a decrease in radial stiffness with increased distance from proximal end of the luminal stent as a consequence of at least one of an increase in the length of the struts of the radially-expandable stent components and a decrease in thickness of the struts of the radially-expandable stent components with increasing distance from the proximal end of the luminal stent. In another version, luminal stent exhibits both a decrease in axial stiffness as a consequence of a decrease in the number of bridges spanning the radially-expandable stent components with increasing distance from proximal end of luminal stent, and a decrease in radial stiffness with increased distance from proximal end of the luminal stent as a consequence of both an increase in the length of struts of radially-expandable stent components and a decrease in thickness of the struts of radially-expandable stent components with increasing distance from proximal end of luminal stent.

Diminishment of either or both of axial stiffness and radial stiffness can be progressive or stepped. Whether progressive or stepped, diminishment of either axial or radial stiffness, in specific embodiments, will not be interrupted by increases of that axial or radial stiffness along that progression. For example, a progression of diminished radial stiffness can be continuous or stepped along a series of radially-expanding stents, despite the fact that they are linked by a luminal graft or a polymeric coating that does not have a radial stiffness that also progressively diminishes.

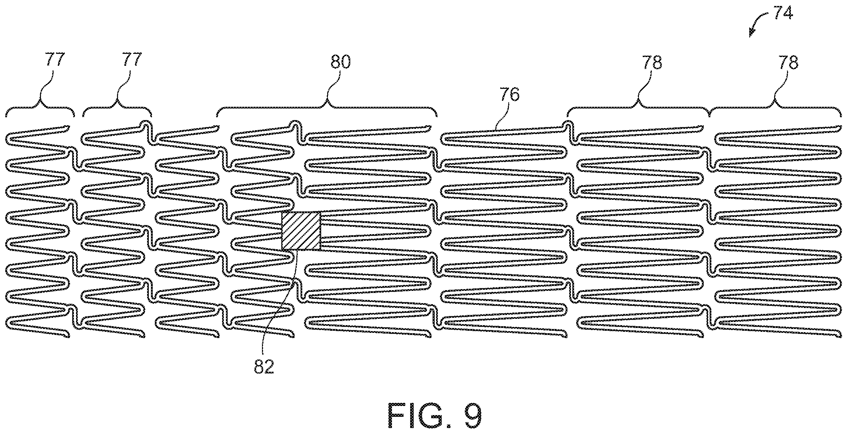

In one version, at least one of the axial stiffness and the radial stiffness of the luminal stent is stepped. For example, in one version, luminal stent 62 includes incremental juncture 64, at which the number of bridges 66 between radially-expanding stent components 68 changes, as shown in FIG. 8, whereby the axial stiffness of luminal stent 62 changes in an increment between proximal end 70 and distal end 72 of luminal stent 62 as a consequence of a reduction in the number of bridges 66 between radially-expandable stent components 68 with increasing distance from proximal end 70 of luminal stent 62. In another version, radial stiffness of luminal stent 74 decreases in steps by either or both an increase in the length of struts 76 of immediately proximal radially-expandable stent components 77 and immediately distal radially-expandable stent components 78, as shown in FIG. 9, and a decrease in thickness (or "width W," as described above with respect to FIG. 7B) of struts of immediately proximal and distal radially-expandable stent components, either of which can be employed to establish juncture 80 between radial stiffness proximal to juncture 80 and radial stiffness distal to juncture 80. A luminal stent can include a plurality of incremental junctures in axial stiffness, radial stiffness, or both, wherein axial or radial stiffness of the luminal stent is constant proximal to the most proximal juncture, between junctures, and distal to the most distal juncture. Radial markers 82 can be fixed at luminal stent 74, such as at junctures 80 of luminal stent 74, as is also shown in FIG 9.

Another version is directed to a luminal stent assembly that includes a luminal stent, such as described above, having a plurality of radially-expandable stent components, each radially-expandable stent component having a proximal end and distal end, at least one of the stent components including struts, wherein the struts include opposite ends and are joined to each other at the respective opposite ends, thereby forming proximal apices and distal apices, the radially-expandable stent components being arranged in relative proximal and distal relationship to each other, and a plurality of bridges linking immediately proximal and distal radially-expandable stent components to each other, thereby forming luminal stent and defining a continuous lumen, and a proximal end and a distal end of luminal stent, wherein at least one of axial stiffness and radial stiffness of the luminal stent decreases from proximal end to distal end of luminal stent. In this version, the luminal stent assembly further includes at least one of a luminal graft component and a polymer coating at the luminal stent.

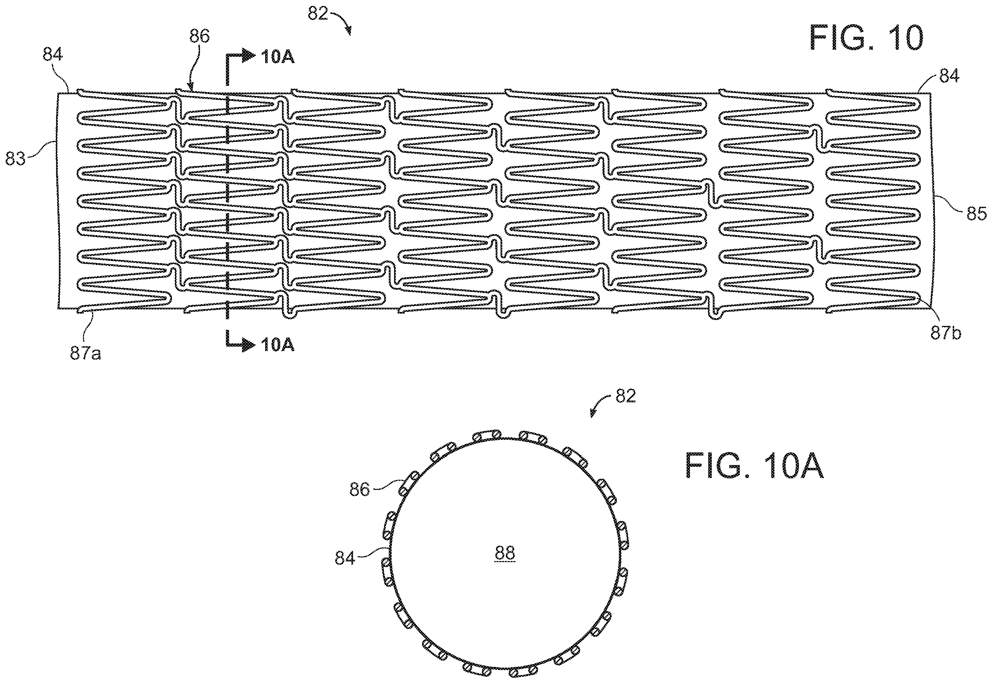

For example, as shown in FIG. 10, luminal stent assembly 82 includes luminal graft component 84 at luminal stent 86. Luminal stent assembly includes proximal end 83 and distal end 85. Luminal stent 86 includes proximal end 87a and distal end 87b. As can be seen in FIG. 10, the number of bridges between radially-expandable stent components of luminal stent 86 decreases with increasing distance from proximal end 87a to distal end 87b of luminal stent 86. FIG. 10A is a cross-section of FIG. 10 taken along line 10A-10A. As shown therein, luminal graft component, in this embodiment, is within lumen 88 defined as luminal stent 82. Although not necessarily so in other versions, luminal graft component 84 extends distally from distal end 87b of luminal stent 80

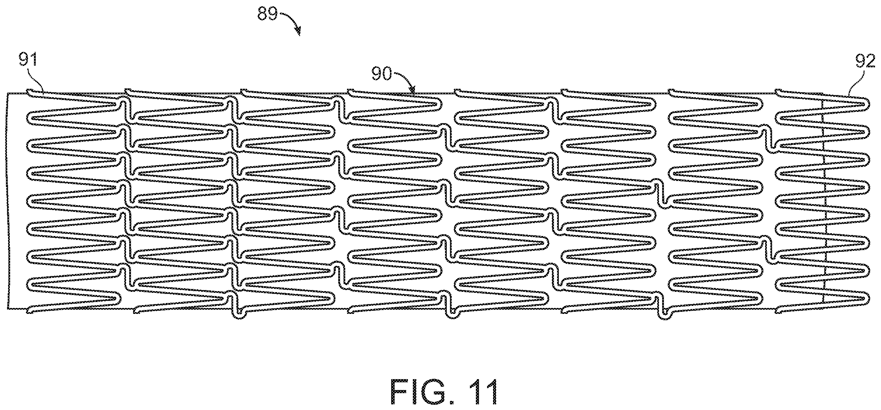

In another versions, shown in FIG. 11, luminal stent assembly 89 includes luminal stent 90 having proximal end 91 and distal end 92, wherein a portion of luminal stent 90 is bare at distal end 92. In another version, not shown, the luminal stent is completely covered on the outside by a luminal graft component.

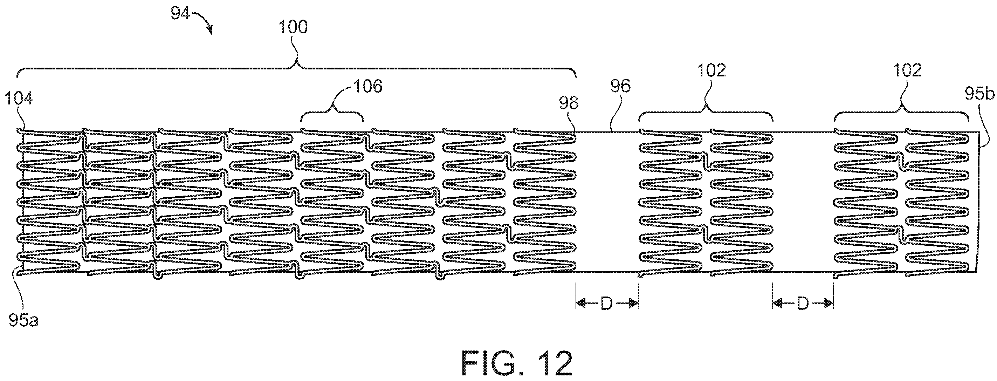

In another version, shown in FIG. 12, luminal stent assembly 94 has proximal end 95a and distal end 95b, and includes luminal graft component 96 extending distally from distal end 98 of luminal stent 100, and further includes at least one radially-expandable stent 102 at luminal graft component 96 and distal to luminal stent 100. In one specific version, the axial stiffness of luminal stent 100 decreases with increasing distance from proximal end 104 to distal end 98 of luminal stent 100 with increasing distance from luminal stent 100 as a result of a decreasing number of bridges from, for example, eight to four to two with increasing distance from proximal end 95a to distal end 95b. The axial stiffness can also decrease as a consequence of increasing distance D between luminal stent 100 and distal stent 102, and between distal stents 102. In one specific embodiment, the radial stiffness of luminal stent 100 decreases from proximal end 104 to distal end 98 of luminal stent 100. In one such version, the radial stiffness of luminal stent assembly 94 decreases with increasing distance from proximal end 104 of luminal stent to distal end 98 of luminal stent 100. In another version, the radial stiffness of luminal stent 100 is constant from proximal end 104 to distal end 98 of luminal stent 100. In this embodiment, the radial stiffness of radially-expandable stent 102 distal to luminal stent 100 is less than that of at least a portion of radially-expandable stent components 106 of luminal stent 100. In one specific version the radial stiffness of radially-expandable stent 102 distal to luminal stent 100 is less than that of most distal radially-expandable stent component 106 of luminal stent 100. In another version, the radial stiffness of the radially-expandable stent 102 is less than that of the luminal stent 100 at proximal end 104 of luminal stent 100.

It is to be understood that the radial stiffness of radially-expandable stent 102 can include resistance to radial expansion from a radially-collapsed position, or resistance to radial collapse from a radially-expanded position, or both. In addition, at least one of the radially-expandable stents 102 distal to luminal stent 100, shown in FIG. 12, can include the same material or composition as that of luminal stent 100, or may be of a different material or composition than that of luminal stent 100.

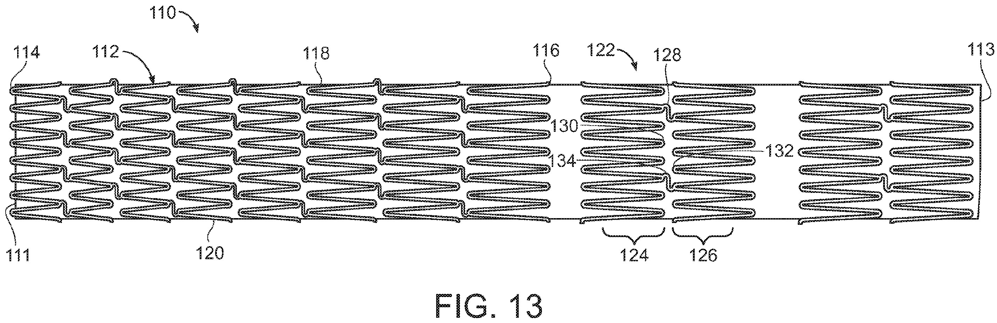

In another version, shown in FIG. 13, luminal stent assembly 110 has proximal end 111 and distal end 113, and includes luminal stent 112 having proximal end 114 and distal end 116. Luminal graft component 118 extends distally from distal end 116 of luminal stent 114. Struts 120 of luminal stent 112 become progressively longer from proximal end 144 to distal end 116 of luminal stent 112. Radially-expandable stent 122 is distal to distal end 116 of luminal stent 112 and includes two radially-expandable stent subcomponents 124, 126. Each stent subcomponent 124, 126 includes struts 128 that define proximal apices 130 and distal apices 132 and are joined by bridge 134. It is to be understood that, in alternative versions, more than one radially-expandable stent can be employed. It is also to be understood that, in versions where at least one radially expandable stent 122 distal to distal end 116 of luminal stent 112 includes radially-expandable stent components 124, 126, that more than two radially-expandable stent subcomponents can be employed in each radially-expandable stent. In one specific version shown in FIG. 13, bridge 134 extends from a distal apex 130 of a most-proximal radially-expandable stent subcomponent 124 to a proximal apex 132 of a most distal radially-expandable stent subcomponent 126 of radially-expandable stent 122

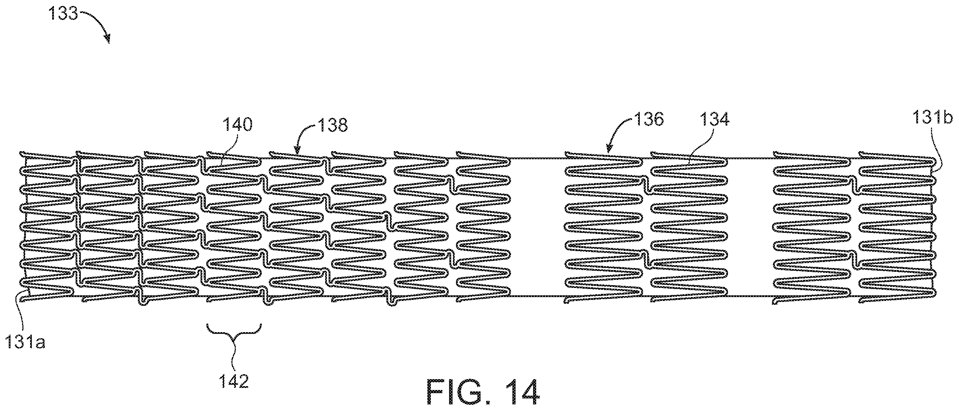

It is to be understood further that struts of the radially-expandable stent distal to the luminal stent can have a length different than those of the radially-expandable stent components of the luminal stent. For example, as shown in FIG. 14, luminal stent assembly 133 having proximal end 131a and 131b, includes struts 134 of radially-expandable stent 136 distal to luminal stent 138 that can be longer than struts 140 of radially-expandable stent components 142 of luminal stent 138, thereby causing the radial stiffness of radially-expandable stent 136 distal to luminal stent 138 to be less than that of radially-expandable stent components 142 of luminal stent 138.

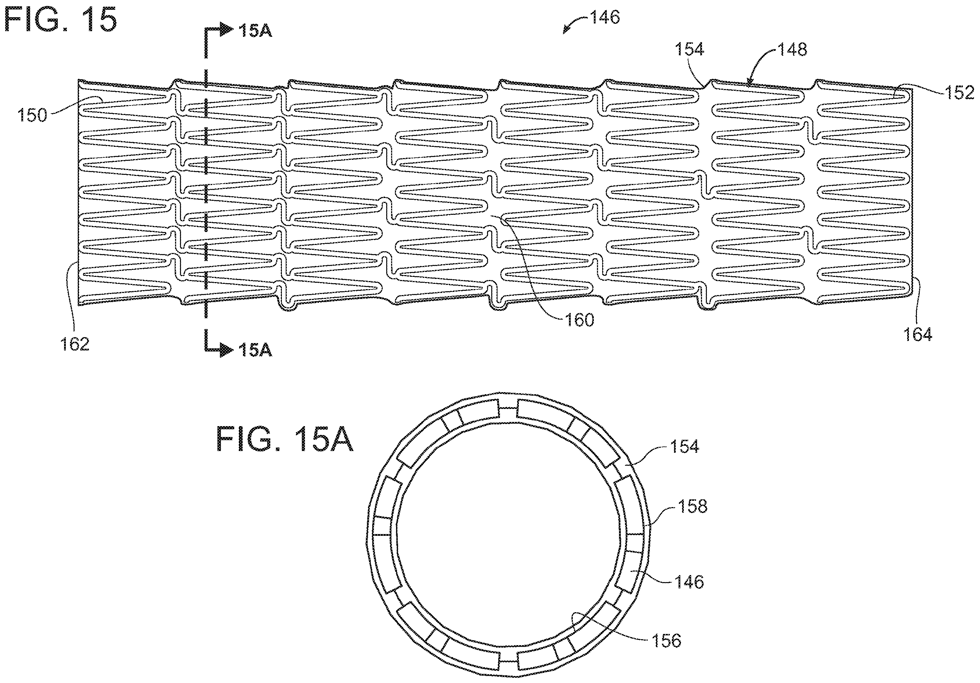

In yet another version, shown in FIGs. 15 and 15 A, luminal stent assembly 146 includes luminal stent 148 having proximal end 150 and distal end 152. Luminal stent assembly 146 further includes polymer coating 154. As can be seen in FIG. 15 A, which is a cross section of luminal stent assembly 146 taken along line 15A-15A of FIG. 15, polymeric coating 154 coats outside surface 158 of luminal stent 146 and inside surface 156 of luminal stent 146. In other versions, polymeric coating 154 may coat only one of outside surface 158 and inside surface 156 of luminal stent 148.

Polymer coating 154 includes at least one layer of polymer. In one version, polymeric coating includes at least one member of the group consisting of polytetrafluoroethylene (PTFE), such as expanded PTFE (ePTFE), polyethylene terephthalate, eSPUN PTFE, FEP, PU (polyurethane), silicone, ePTFE with PU bond catalyst, and ePTFE with FEP bond catalyst. In a specific embodiment, polymeric coating 154 includes ePTFE. In a still more specific embodiment, polymeric coating 154 is ePTFE.

In another version, polymeric coating 154 coats inside surface 156 of luminal stent 146 and outside surface 158 of luminal stent 146, and includes a plurality of layers on at least one of inside surface 156 of luminal stent 146 and outside surface 158 of luminal stent 146. In a specific version, polymeric coating 154 includes a plurality of polymer layers on both inside surface 156 and outside surface of luminal stent 158. In yet another embodiment, there are more layers of polymeric coating at at least one of distal end 152 and proximal end 150 of the luminal stent 148 than between distal end 152 and proximal end 150 of luminal stent 148. In yet another version, there are more layers of polymer of polymeric coating 154 at both distal end 152 and proximal end 150 of luminal stent 146 than between distal end 152 and proximal end 150 of luminal stent 146.

In yet another version, polymeric coating 154 on inside surface 158 and outside surface 158 of luminal stent 146 is continuous. In one such version, luminal stent 146 defines openings 160 and polymeric coating 154 seals openings. In still another version, the total thickness of polymeric coating 154 on inside surface 156 of luminal stent is greater than the total thickness of polymeric coating 154 on outside surface 158 of luminal stent 148. In one such version, the total thickness of polymeric coating 154 on inside surface 156 of luminal stent 148 is in a range of between about 0.001 mm and about 0.1 mm. In another such version, the total thickness of polymer coating 154 on outside surface 158 of luminal stent 148 is in a range of between about 0.001 mm and about 0.1 mm. In a specific version, the total thickness of polymer coating 154 on inside surface 156 of luminal stent 148 can range from 0.001mm to 0.1mm, and the total thickness of polymer coating 154 on outside surface 158 of luminal stent 148 can range from 0.001 mm to 0.15 mm.

In still another version, not shown, polymeric coating 154 covers inside surface 156 of luminal stent 148, outside surface 158. In one specific version, shown in FIGs. 15 and 15 A, both outside surface 156 and inside surface 156 of luminal stent. Optionally, and not shown, a graft component can be present and cover polymer coating 154 on outside surface 158 of luminal stent 148. In at least one version, proximal end 162 and distal end 164 of polymeric coating 154 are fabricated to prevent preferential radial expansion between proximal end 150 and distal end 152 of luminal stent 148. Such radial expansion, also known as "dog-boning," can be largely or completely avoided, at least in one version, by forming polymeric coating 154 with more polymeric layers at proximal end 162 and at distal end 164 of polymeric coating 154 than between proximal end 162 and distal end 164 of polymeric coating 154. A benefit of this particular version is that, during implantation, the likelihood of damage to the peripheral branch artery in which luminal stent assembly 146 is being implanted is diminished during radial expansion.

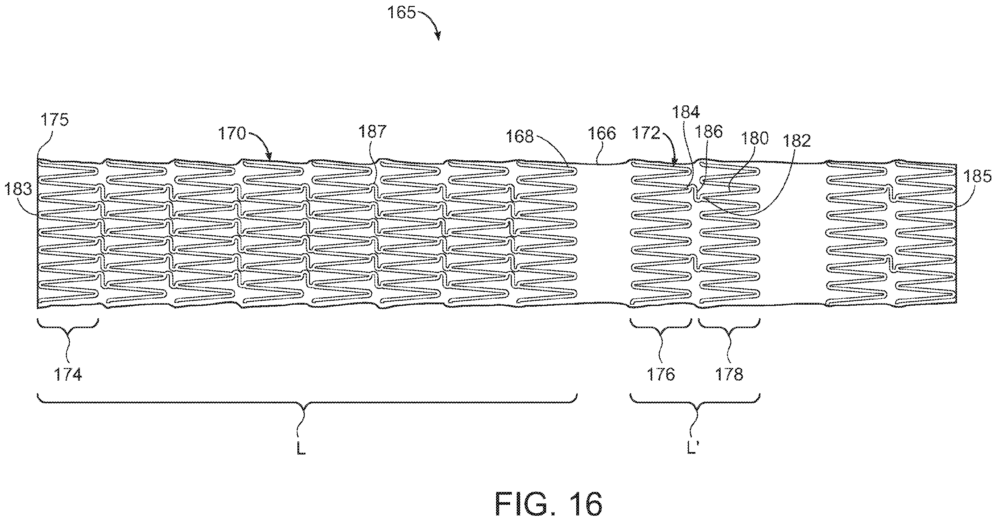

In one version, as shown in FIG. 16, luminal stent assembly 165 includes polymeric coating 166 that extends distally from distal end 168 of luminal stent 170. Radially-expandable stent 172 is distal to luminal stent 170 and is also at polymeric coating 166. In this version, the radial stiffness of radially-expandable stent 172 is less than that of at least a portion of luminal stent 170. As previously explained, the radial stiffness of radially expandable stent 172 can include resistance to radial expansion from a radially-collapsed position, resistance to radial collapse from a radially-expanded position, or both. Further, and as also previously described, at least one of radially-expandable stents 172 distal to distal end 168 of luminal stent 170 can be formed of the same material composition as that of luminal stent 170. Although not shown, the most proximal of radially-expandable stent components 174 at proximal end 175 of luminal stent 170 can be flared. In another version, and as also explained above, at least one of radially-expandable stents 172 distal to distal end 168 of luminal stent 170 can include two radially-expandable stent subcomponents 176, 178, each radially-expandable stent component 176, 178 including struts 180 that define proximal and distal apices 182, 184, wherein radially-expandable stent subcomponents 176, 178 are joined by at least one bridge 186. In such a version, at least one bridge 186 of radially-expandable stent 172 can extend from distal apex 184 of most-proximal radially-expandable stent subcomponent 176 to proximal apex 182 of most-distal radially-expandable stent subcomponent 178 of radially-expandable stent 172. In this embodiment, axial stiffness of luminal stent assembly 165 is decreased at distal end 185 of luminal stent assembly 165 relative to proximal end 183 of luminal stent assembly 165 because there are fewer bridges 186 linking radially-expandable stent subcomponents 176, 178 of radially-expandable stent 172 than there are bridges 187 linking radially-expandable stent subcomponents 174 of luminal stent 170. Axial stiffness of luminal stent assembly 165 is also lower at distal end 185 than at proximal end 183 of luminal stent assembly because luminal stent 170 has a greater longitudinal length L than the length L' of any radially expandable stents 172 distal to distal end 168 of luminal stent 170.

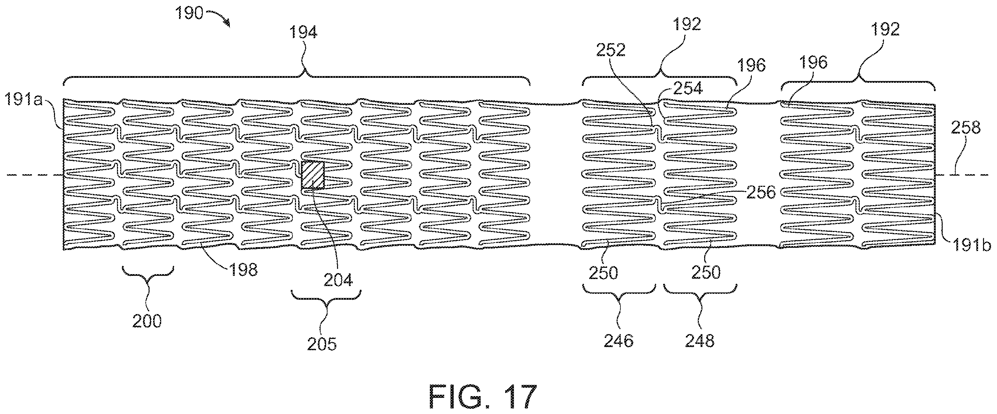

In another version, struts of radially-expandable stents can have a length different than those of at least a portion of radially-expandable stent components of luminal stent. For example, as shown in FIG. 17, luminal stent assembly 190 has proximal end 191a and 191b, and includes radially-expandable stents 192 distal to luminal stent 194, wherein struts 196 of luminal stent 192 are longer than at least a portion of struts 198 of radially-expandable stent components 200 of luminal stent 194. In one version, at least one of axial and radial stiffness of luminal stent 194 is stepped, wherein luminal stent 194 further includes an incremental juncture, as described with respect to earlier versions described above, whereby at least one of axial stiffness and radial stiffness of luminal stent 194 changes in at least one incremental juncture. In a more specific version, luminal stent 194 includes a plurality of incremental junctures, whereby radial stiffness of radially-expandable stent components 200 of luminal stent 194 decreases with increasing distance from proximal end 202 of luminal stent 194. In yet another version, and as further described above with respect to earlier versions, luminal stent assembly 194 can include at least one radiopaque marker 204 at luminal stent 190, such as at at least one of a plurality of incremental junctures 205 of luminal stent 190.

At least one radially-expandable stent 192 distal to distal end 244 of luminal stent 194 includes two radially-expandable stent subcomponents 246, 248, each radially-expandable stent subcomponent 246, 248 including struts 250 that are joined to define distal apices 252 and proximal apices 254, and wherein radially-expandable stent subcomponents 246, 248 are joined by at least one bridge 256. In at least one version, bridges 256 extend transversely to longitudinal axis 258 of luminal stent assembly 190. In another version, not shown and as previously described, struts of radially-expandable stent subcomponents have at least one of a length greater, or a thickness less than those of radially-expandable stent components 200 of the luminal stent 198.

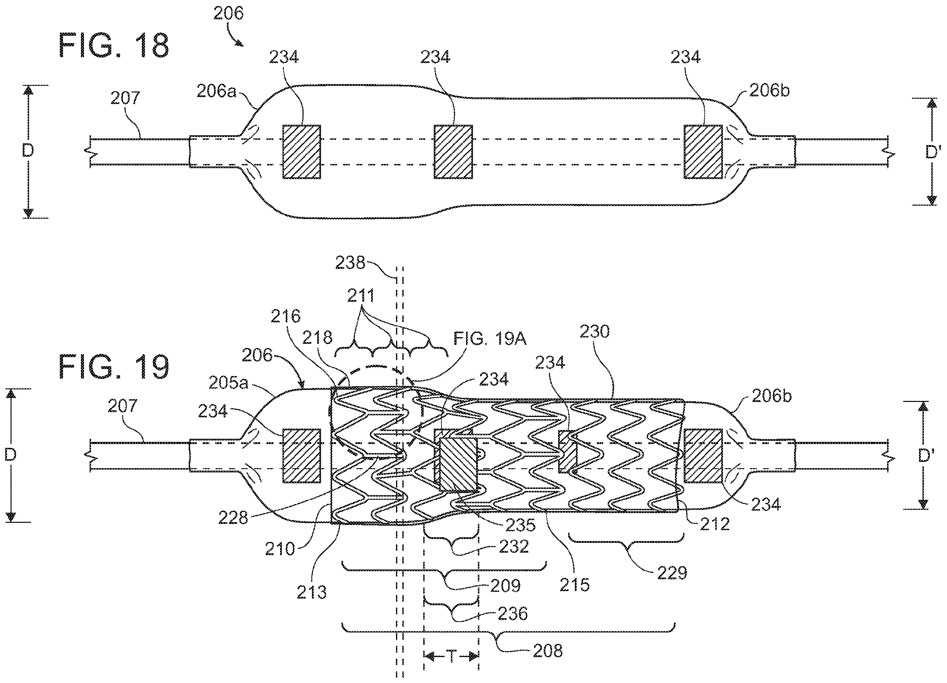

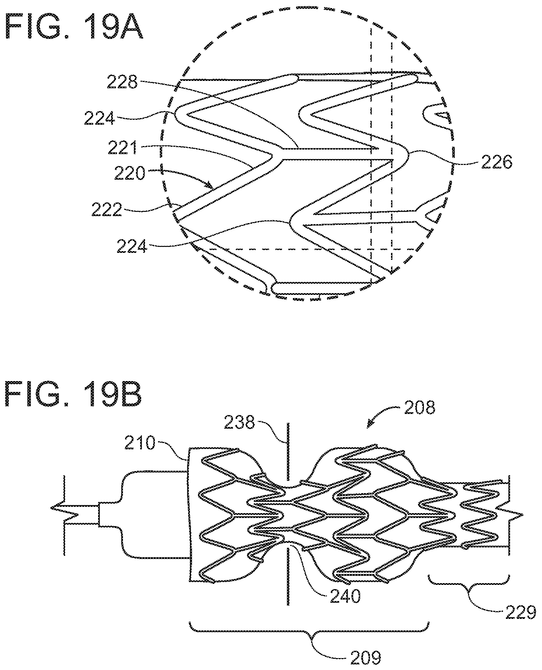

In still another version, luminal stent assembly includes balloon 206, shown alone and inflated in FIG. 18. Balloon 206 is linked to a fluid source, not shown, by catheter 207. Balloon 206 has a greater diameter D at proximal end 207 than diameter D' at distal end 210 when inflated. As shown in Fig. 19, balloon 206 is located within luminal stent 208. Luminal stent assembly 208 has proximal end 210 and distal end 212, and is made up, at least in part, of luminal stent 209 and a plurality of radically-expandable stents 211 distal to luminal stent. Luminal stent 209 includes proximal end 213 and distal end 215, and radially-expandable stent components 214, each radially-expandable stent component 214 having proximal end 216 and distal end 218, wherein at least one of stent components 214 includes struts 220. As shown in FIG. 19 A, struts 220 include opposite ends 221, 222 and are joined to each other at respective opposite ends, thereby forming proximal apices 224 and distal apices 226. Radially-expandable stent components 214 are arranged in relative proximal and distal relationship to each other, and are linked by plurality of bridges 228 between immediately proximal and distal radially-expandable stent components 214 to each other. Luminal stent assembly 208 also includes radially-expandable stents 229 distal to luminal stent 209, at least one of a luminal graft component (not shown) and polymeric coating 230 at luminal stent, and balloon 206 within the luminal stent assembly 208 when in a collapsed position (not shown). Balloon 206 has a greater diameter D at proximal end 207 of luminal stent when inflated. As described above, diameters D, D' of balloon 206 can be stepped with distance between proximal end 207 and distal end 210, whereby balloon 206 includes proximal diameter D, distal diameter D', and transition area 232 between proximal diameter D and distal diameter D'. Balloon 206 can be fabricated of any suitable material, such as a semi-compliant material. Radiopaque markers 234 are fixed to balloon 206, and marker 235 is fixed to luminal stent 209 at junction area 236 of luminal stent assembly 208. Transition area 232 of balloon 206 has transition length T.

In this version, axial stiffness of luminal stent 209 decreases from proximal end 213 and to distal end 215 by a suitable means, such as by a decrease in the number of bridges 228 spanning radially-expandable stent components 211. Radial stiffness can also decrease with increasing distance from proximal end 213 of luminal stent 209 by a suitable means, such as by an increase in the length of struts 228 of radially-expandable stent components 211, and a decrease in thickness of struts 228 of radially-expandable stent components 211 with distance from proximal end 213 of luminal stent 209. In one such version, at least one of axial stiffness and radial stiffness of luminal stent 209 is stepped, wherein luminal stent 209 further includes incremental junction 236, whereby radial stiffness of luminal stent 209 changes in at least one increment. In one such version, luminal stent 209 includes a plurality of incremental junctions (not shown), wherein at least one of the axial stiffness and the radial stiffness of luminal stent 209 decreases with increasing distance from proximal end 213 of luminal stent 209.

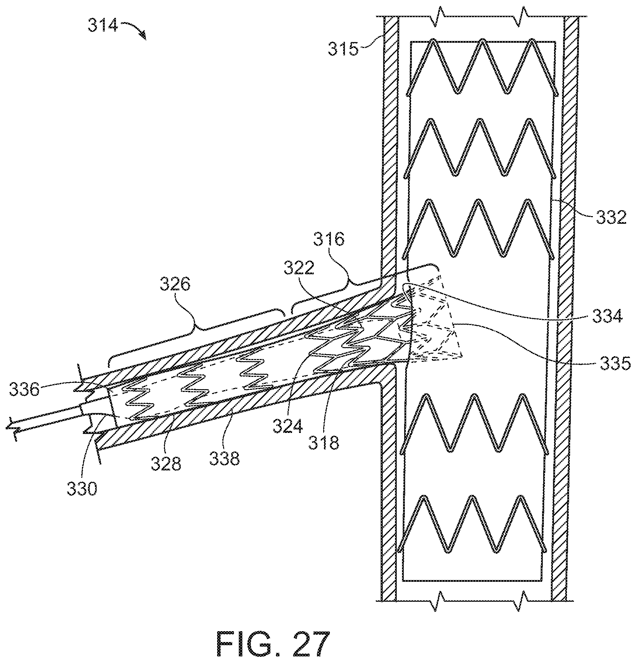

As shown in FIG. 19, proximal end 206a of balloon 206 is proximate to proximal end 210 of luminal stent assembly 208 and distal end of balloon 206b is proximate to distal end 212 of luminal stent assembly 208. Upon inflation, balloon 206 exerts greater radial expansive force against an inside surface of luminal stent at proximal end 210 of luminal stent assembly 208 than at distal end 212 of luminal stent assembly 208, thereby causing luminal stent to have a greater outside diameter at proximal end 210 than at distal end 212, absent some constricting force at luminal stent 209, such as would be applied at a fenestration in a fenestrated stent graft 238 in which luminal stent assembly 208 has been placed, as shown in phantom in FIG. 19, and as shown in FIGs. 27, 28 and 31, described infra. For example, as shown in FIG. 19B, inflation of balloon 206 is within fenestration 240 of fenestrated stent graft 238 will cause constriction of luminal stent 209 at fenestration 240 relative to the remainder of luminal stent 209 on either side of fenestration 240. In one version, additional layers of polymer are included at distal end 206b of balloon 206 to prevent excessive radial expansion of distal end 212 of the luminal stent assembly 208 when the balloon is not protruding from the distal end of the stent, thereby keeping the distal end of the stent and the balloon from overdilating and, consequently, preventing damage to the vascular branch into which the luminal stent assembly 208 has been implanted.

In one version, radially-expandable stents 229 distal to luminal stent 209 in this embodiment has at least one of an axial stiffness and a radial stiffness less than that of luminal stent 209. In one specific version, at least one radiopaque marker 234 is fixed to luminal graft component (not shown) or polymeric coating 230 between luminal stent 209 and radially-expandable stent 229 distal to luminal stent 209. In this version, in one variation, luminal stent assembly 208 includes a variation in radial stiffness at luminal stent 230, whereby radial stiffness is stepped, thereby forming junctures 238 at luminal stent 209, and further including a second radiopaque marker 234 at at least one incremental juncture 236 of luminal stent 209.

As discussed above with respect to earlier versions, radial stiffness of the radially-expandable stent 229 can be resistance to radial expansion from a radially collapsed position. In another version, the material in the radially-expandable stents 229 distal to luminal stent can be fabricated of the same material as that of luminal stent 209. In such a version, not shown, the most proximal of the radially-expandable stent components can be flared at the proximal end of the luminal stent upon implantation, as will be described below.

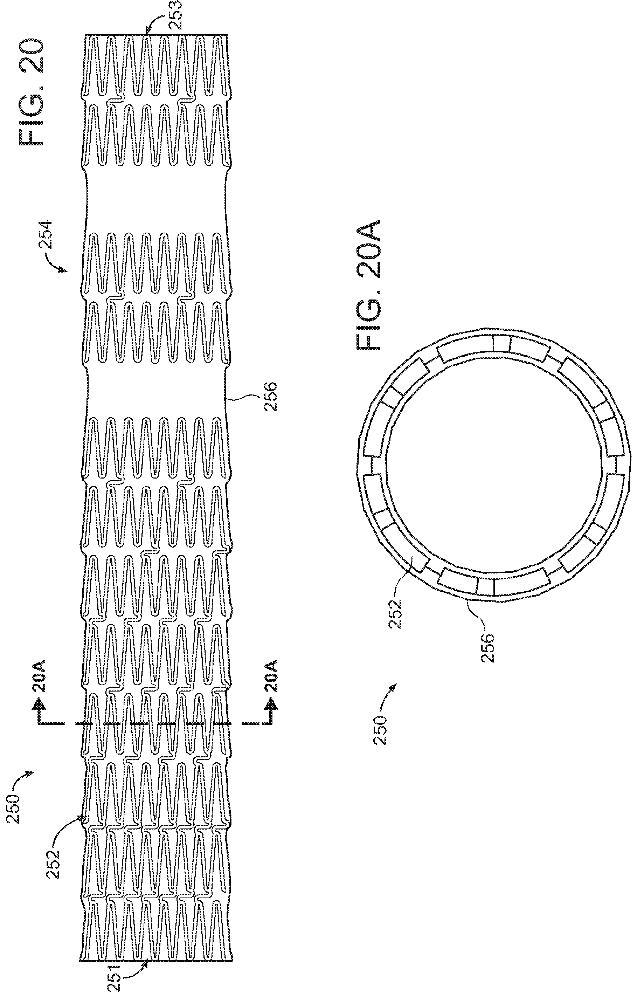

Another version, the invention is a luminal stent assembly that includes a luminal stent and a polymeric coating at the luminal stent, either or the combination of which is collectively represented. In this version, shown in FIGs. 20 and 20A, luminal stent assembly 250 has proximal end 251 and distal end 253, and includes luminal stent 252 having a plurality of radially-expandable stent components 254, as similarly indicated in FIG. 12. In FIGs. 20 and 20B, however, luminal graft component 96 of FIG. 12 is substituted with polymeric coating 256 that lines inside surface 258 and outside surface 269 of luminal stents 252.

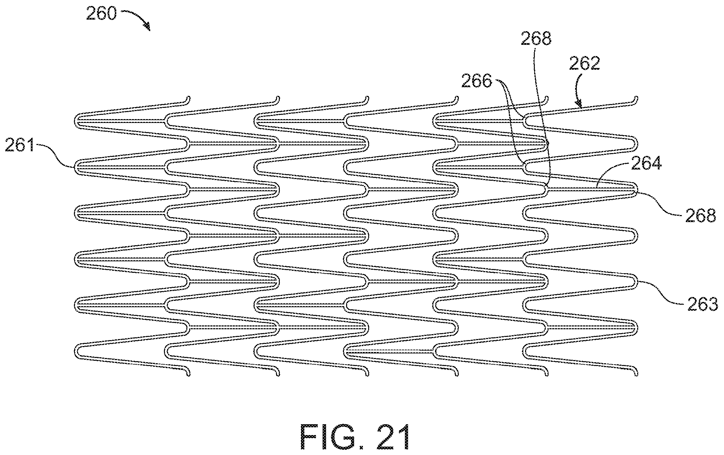

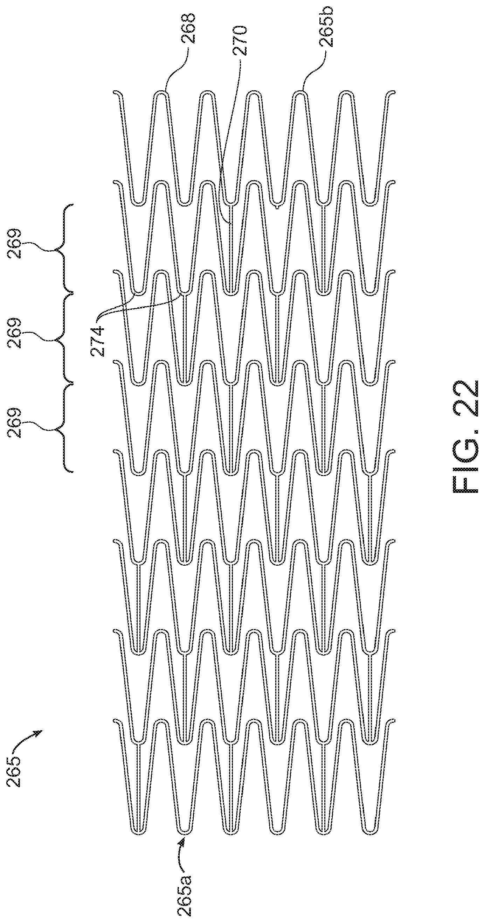

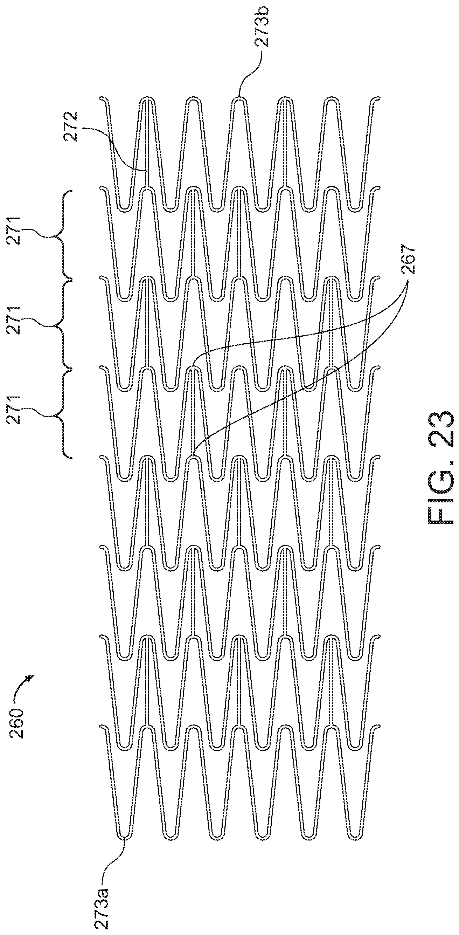

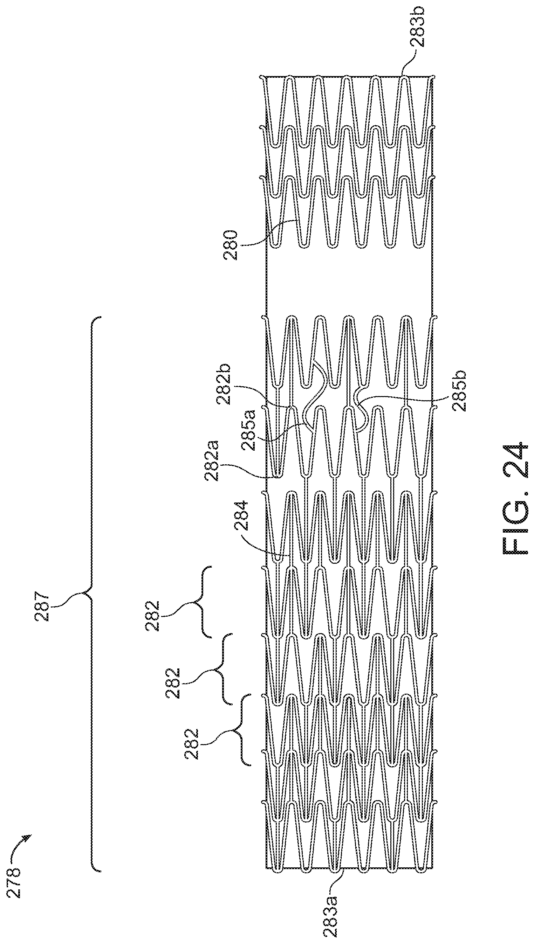

In one variant of the embodiment of FIG.1, shown in FIG. 21, luminal stent 260 has proximal end 261 and distal end 263, and includes at least a portion of radially-expandable stent components 262 that are nested. In this embodiment, radially-expandable stent components 262 can be linked by bridges 264 extending between respective proximal apices 266 and respective distal apices 268 of adjacent radially-expandable stent components 262. In another embodiment, such as shown in FIG. 22, luminal stent 265 includes proximal end 265a and distal end 265b, and is made up of radially-expandable stent components 269 and bridges 270 extending between only proximal apices 274. In yet another embodiment, shown in FIG 23, lumina stent 273 has proximal end 273a and distal end 273b, and includes radially-expandable stent components 271 linked by bridges 272 only between distal apices 267 of proximate proximal and distal radially-expandable stent components 271. In still another embodiments, such as is shown in FIG. 24, in luminal stent assembly 278, which includes proximal end 283a and distal end 283b, a combination of proxial apices 282a and distal apices 282b of nested radially-expandable stent component 282s can be linked. Optionally, the number of bridges 284 between radially-expandable stent components 282 diminishes with distance from the proximal end of the luminal stent. In such an embodiment, or in another embodiment, luminal stent assembly 278 can include distal stents 280 that are nested, but wherein radially-expandable stent components 282 of luminal stent 287 may or may not be nested. More specifically, in one embodiment, such as is seen in FIG. 24, the degree of nesting of radially-expanding stent components 282 decreases with increasing distance from proximal end 283 toward distal end 283b of luminal stent assembly 278, thereby decreasing the axial stiffness (or "bending stiffness") of luminal stent assembly 278 with increasing distance from proximal end 283. S-shaped bridges 285a and 285b can bridge struts of radially-expandable stent component 282 of luminal stent 287, either between radially-expandable stent components that are not nested, as shown in FIG. 24, or between struts of nested immediately distal and proximal radially-expandable stents, not shown, to thereby control at least one of axial stiffness and radial stiffness.

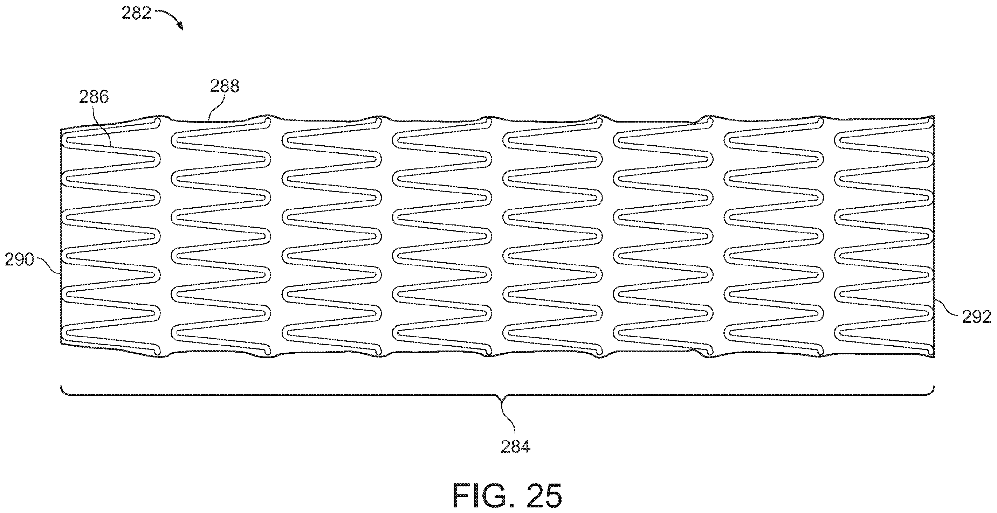

In another version, shown in FIG. 25, luminal stent assembly 282 includes luminal stent 284 of radially-expandable stents 286 that are not nested, wherein the radially-expandable stents 286 are linked by a polymeric coating 288. It is to be understood that, in this version, either or both the axial stiffness and the radial stiffness can decrease with increasing distance from proximal end 290 to distal end 292 of luminal stent assembly 282, either as a consequence of a decrease of either or both of respective decreasing axial and radial stiffness of radially-expandable stent 286 relative to each other.

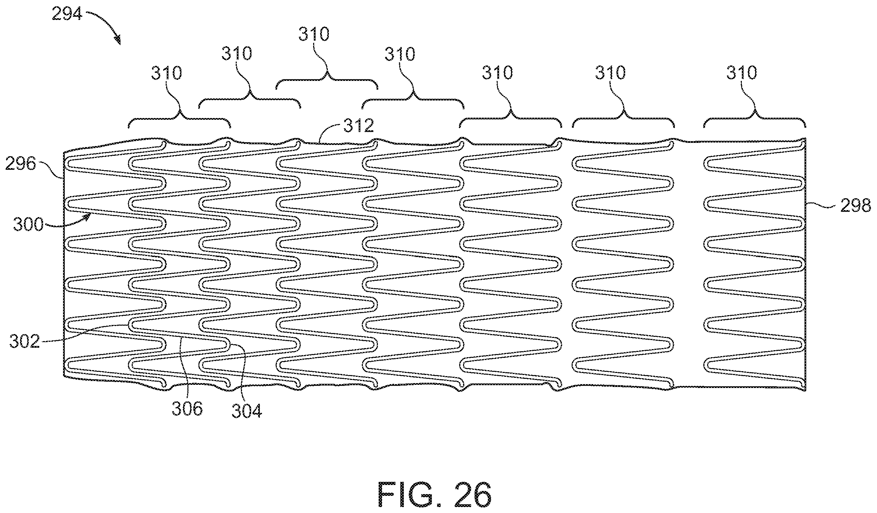

In still another version, shown in FIG. 26, luminal stent assembly 294 includes proximal end 296 and distal end 298, and a plurality of radially-expandable stents 300. Each radially-expandable stent 300 includes proximal end 302 and distal end 304. Radially-expandable stents 300 include struts 306. Struts 306 include opposite ends that are joined to each other to form proximal and distal apieces. Radially-expandable stents 300 are in relative proximal and distal relationship to each other. The radial stiffness of each radially expandable stent 310 is less than that of each radially-expandable stent 310 proximal to it, and greater than that of each radially-expandable stent 310 distal to it. Radially expandable stents 310 are linked by at least one of a luminal graft component (not shown) and polymeric coating 312. Radially-expandable stents 310 are decreasingly nested with each other with increasing distance from proximal end 296 to distal end 298.

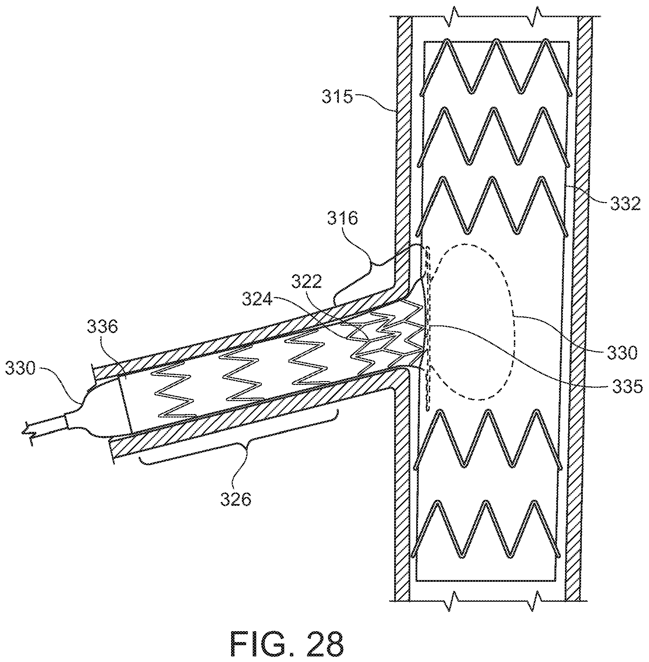

As shown in FIGs. 27 and 28, luminal stent assembly 314, shown partially implanted within a branched artery 315, includes luminal stent 316 having a plurality of radially-expandable stent components 318 and plurality of bridges 320 linking immediately proximal and distal radially-expandable stent components 318 to each other. Radially-expandable stent components 318 each include struts 320 wherein the struts include opposite ends that are joined to each other at respective opposite ends, thereby forming proximal apices 322 and distal apices 324, like apices previously described. Radially-expandable stent components 318 are arranged in relative proximal and distal relationship to each other and linked by bridges 320 at distal apices 324 and proximal apices 322. Radially-expandable distal stents 326 are located distally to luminal stent 316. At least one of a luminal graft component (not shown) and polymeric coating 328 are at luminal stent. Balloon 330 extends within luminal stent 316 when luminal stent 316 is in a collapsed position, as shown in FIG. 27, and has a greater diameter at a proximal end than at a distal end when inflated, as shown at FIG. 28. Fenestrated stent graft 332 defines at least one fenestration 334, wherein luminal stent 316 at proximal end 335 has a diameter less than fenestration 334 when in a collapsed position, shown in FIG. 27, and is expandable to a diameter that fixes luminal stent 316 within fenestration 334, whereby distal end 336 of luminal stent assembly 314 extends radially from fenestrated stent graft 332 into branch 338 of branched artery 315, as shown in FIG. 28.

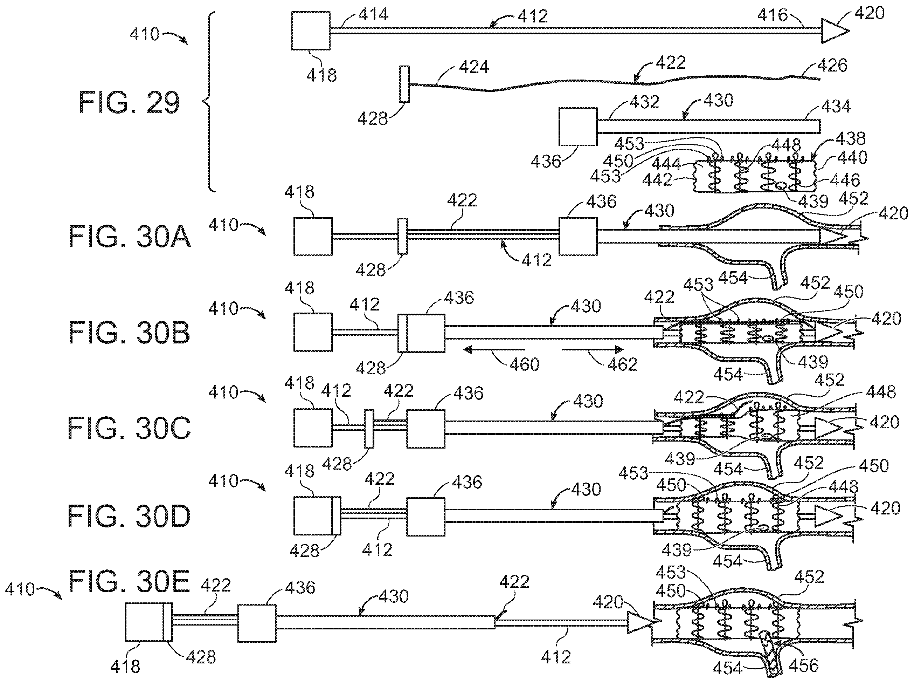

FIG. 29 is an exploded side view of another version. Stent graft delivery system 410 as described below is based on the teaching of PCT/US2018/019342, filed February 23 2018. More specifically, stent graft delivery system 410 includes guidewire catheter 412 having proximal end 414 and distal end 416. Proximal handle 418 is fixed to proximal end 414 of guidewire catheter 412. Nose cone 420 is fixed to distal end 416 of guidewire catheter 412. Wire 422 includes proximal end 424 and distal end 426. Wire 422 can be fabricated of a suitable material, such as is known in the art, including, for example, Nitinol or some other shape memory alloy, or stainless steel. Wire 422 is sufficiently flexible not to injure the patient during advancement to an aortic aneurysm of a patient. Wire handle 428 is fixed at proximal end 424 of wire 422. Introducer sheath 430 includes proximal end 432 and distal end 434, and distal handle 436 is fixed to proximal end 432 of introducer sheath 430. Stent graft 438 includes proximal end 440, distal end 442, luminal graft component 444, stents 446 distributed along luminal graft component 444, and ligatures 448, as described in PCT/US2018/019342.

FIG. 30A is an assembled side view of stent graft delivery system 410 shown in FIG. 30, wherein stent graft 438 has been loaded within distal end 434 of introducer sheath 430, and radially constricted, at least in part, by wire 422 threaded through loops 450 at ends of ligatures 448, as discussed in PCT/US2018/019342, and through stabilizing anchor loops 453. Stent graft 438 can include fenestration 439. In a method stent graft delivery system 410 is advanced to arterial aneurysm 452 of a patient. In one version, shown in FIG. 30A, introducer sheath 430 is advanced to aneurysm site 452 to thereby place stent graft 438 at arterial aneurysm 452. As can be seen in FIG. 30B, distal handle 436 is retracted in a proximal direction indicated by arrow 460 toward proximal handle 418, thereby retracting introducer sheath 430 from stent graft 438 at aneurysm 452. As can be seen in FIG. 30B, despite retraction of introducer sheath 430, stent graft 438 is maintained in a radially constricted position by wire 422 extending through ligature loops 450 of ligatures 448 traversing struts of stents 446 distributed longitudinally along stent graft 438. It is to be understood, however, that in alternative methods, stent graft delivery system 410 can be advanced within an artery to a position distal to arterial aneurysm 452, wherein stent graft 438 is directed to arterial aneurysm 452 by advancement of proximal handle 418 and wire handle 428 in a distal direction indicated by arrow 462 toward distal handle 436 to thereby direct radially-constricted stent graft 418 from introducer sheath 430 to arterial aneurysm 452.

Following direction of stent graft 438 to a position that spans aneurysm 452, and at least partial rotational and axial alignment of stent graft 438 at aneurysm 452, wire 422 is partially retracted from loops 450 of ligatures and from anchor loops 453. Proximal retraction of wire handle 428 toward proximal handle 418, in the direction indicated by arrow 460, can be seen in FIG. 30C. Continued retraction of wire 422 withdraws wire 422 from all suture loops 450 of ligatures 448 and anchor loops 453, thereby enabling stent graft 438 to fully expand from its radially constricted state, shown in FIG. 30B, to a radially expanded state, shown in FIG. 30D. Stent graft 438 is positioned so that fenestration 439 is properly aligned with arterial branch 454 for subsequent placement of branch prosthesis 456 through fenestration 439 to arterial branch 454. Thereafter, stent graft 438 is fully implanted within aneurysm, and the remainder of stent graft delivery device 410 is retracted from stent graft 438 and the patient, as shown in FIG. 30E, thereby completing treatment of aneurysm site 452 of the patient.

Vascular prostheses implanted by the stent graft systems and methods can be implanted, for example, by transfemoral access. Additional branch prostheses that are directed into the vascular prostheses can be implanted, for example, by supraaortic vessel access (e.g., through the brachial artery), or by transfemoral access, or access from some other branch or branches of major blood vessels, including peripheral blood vessels.

In a method of implanting a stent graft system, a fenestrated stent graft of the stent graft system is delivered to a branched artery in a subject, wherein a fenestration defined by the fenestrated stent graft aligns with the proximal end of the branched artery. A luminal stent or luminal stent assembly is delivered through the fenestration and into an arterial branch, wherein the luminal stent or the luminal stent of the luminal stent assembly has a proximal end at the fenestration of the fenestrated stent graft and a distal end extending radially outward from the fenestrated stent graft, as shown in FIG. 30E. The luminal stent is radially expanded within the fenestration and the arterial branch by inflating a balloon within the luminal stent that has a greater diameter at the proximal end of the luminal stent than at the distal end of the luminal stent, thereby implanting the stent graft system, as represented above in FIGs. 27, 28 and 31.

In one example, the luminal stent of the stent graft system implanted by the method includes, as described in detail above, a plurality of radially-expandable stent components, wherein the radially expandable stent components are joined at respective ends, thereby forming proximal apices and distal apices, the radially expandable stents being arranged in relative proximal and distal relationship to each other to thereby form a luminal stent defining a continuous luminal, a proximal end and a distal end of the luminal stent. The luminal stent in this example also includes a plurality of bridges, each of which link a relatively proximal radially-expandable stent component to an immediately distal radially-expandable stent component, wherein the luminal stent has at least one of an axial stiffness and a radial stiffness that decreases with increasing distance from the proximal end of the luminal stent. In one such example, the axial stiffness of the luminal stent decreases from the proximal end to the distal end of the luminal stent consequent to a decrease in the number of bridges spanning the radially-expandable stent components, and optionally or in the alternative, radial stiffness decreases with increasing distance from the proximal end of the luminal stent by at least one of an increase in the length of the struts of the radially-expandable stent components and a decrease in thickness of the struts of the radially expandable stent components with distance from the proximal end of the luminal stent. All of which is described in detail above.

In another example of a method of implanting a stent graft system, a fenestrated stent graft of the stent graft system, such as is described in detail above, is delivered to a branched artery of a subject, wherein fenestration defined by the fenestrated stent graft aligns with a proximal end of a branched artery of a patient. A luminal stent assembly of a stent graft system is delivered at least partially through the fenestration and into the arterial branch, the luminal stent assembly thereby bridging the fenestrated stent graft and arterial branch, whereby the luminal stent assembly includes a plurality of stents aligned longitudinally and connected by bridges to form a luminal stent. At least one stent distal to the luminal stent has a radial stiffness less than that of the luminal stent. At least one of a luminal graft component and a polymeric coating links the luminal stent and the stent distal to the luminal stent. The luminal stent assembly is radially expanded within the fenestration of the arterial branch by inflating a balloon within the luminal stent assembly, wherein the balloon has a greater diameter at the luminal stent than at the distal stent to the luminal stent when inflated, thereby fixing the luminal stent within the fenestration and implanting the stent graft system. All of which is described in detail above.

In one example of this method, the stent distal to the luminal stent is linked to the luminal stent by the luminal graft component. In another example, the stent distal to the luminal stent is linked to the luminal stent by the polymeric coating. In still another embodiment, the polymeric coating encapsulates the luminal stent and the stent distal to the luminal stent. In still another embodiment, at least one of the stent distal to the luminal stent and the luminal stent defines openings, and the polymeric coating seals the openings and defines a luminal space between the stent distal to the luminal stent and the luminal stent. All of which is described in detail above.

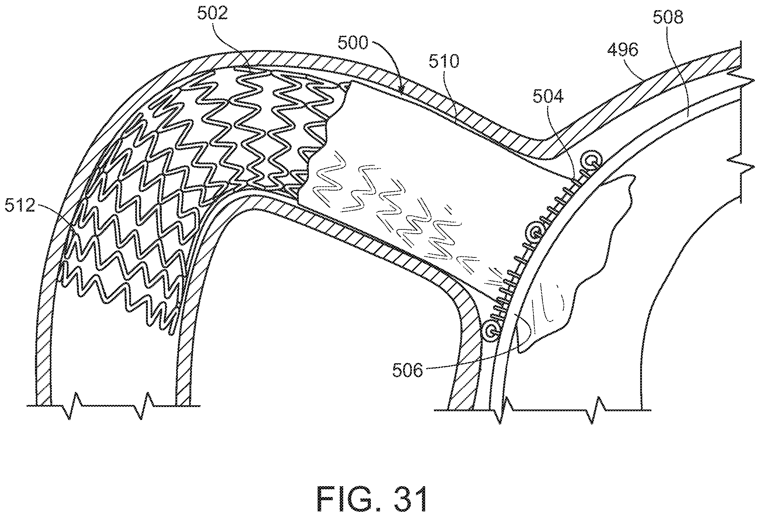

Once radially-expanded within fenestrated stent graft, such as is shown in FIG. 31, luminal stent assembly 500 includes luminal stent 502 that is flared at proximal end 504. Flaring at proximal end 504 can be obtained by inflation of a balloon, as previously described, within luminal stent assembly 500, while proximal end 504 is within fenestration 506 of fenestrated stent graft 508 that has previously been implanted in a branched artery 496 in a subject in order to, for example, secure proximal end 504 of luminal stent assembly 500 within fenestration 506 of the fenestrated stent graft 508. As can be seen in FIG. 31, proximal end 504 of luminal stent assembly 500 is covered by polymeric coating 510, while luminal stent 502 is exposed, or bare, at distal end 512 of luminal stent assembly 500.

In one particular version, each radially-expandable stent component of luminal stent has a longitudinal length in a range of between about 10 mm and about 80 mm, luminal stent has between about 3 and about 40 radially-expandable stent components, at least a portion of which have a longitudinal length in a range of between about 3 mm and about 20 mm, each including between about two and about four struts, although as many as about forty struts can make up each radially-expandable stent component. Also, at least a portion of radially-expandable stent components can have a longitudinal length along a longitudinal axis of luminal stent, of about 2.5 mm, for example, and the distance between radially-expandable stent components each radially-expandable stent component can be about 0.2 mm. The total length of the luminal stent can be, for example, in a range of between about 2 mm and about 5 mm, such as where there are four radially-expandable stent components of the luminal stent, and the number of struts per radially-expandable stent component is six.

## Claims

1. A luminal stent assembly (104), comprising:
a) a luminal stent (100) including,
i) a plurality of radially-expandable stent components (106), each radially-expandable stent component having a proximal end and a distal end, at least one of the stent components including struts, wherein the struts include opposite ends and are joined to each other at the respective opposite ends, thereby forming proximal apices and distal apices, the radially-expandable stent components (106) being in relative proximal and distal relationship to each other, and
ii) a plurality of bridges (28) linking immediately proximal and distal radially-expandable stent components (106) to each other, thereby forming the luminal stent (100) and defining a continuous lumen, a proximal end, and a distal end of the luminal stent (100);
b) at least one of a luminal graft component (96) and a polymeric coating (154) at the luminal stent (100); and
c) at least one stent distal (102) to the luminal stent (100) and linked to the luminal stent (100) by at least one of the luminal graft component (96) and the polymeric coating (154) to thereby form the luminal stent assembly (104), the luminal stent assembly (104) having a proximal end at the luminal stent (100) and a distal end at the stent distal to the luminal stent (100),
wherein axial stiffness of the luminal stent assembly (104) decreases from the proximal end to the distal end of the luminal stent assembly (104) as a consequence of a space between the luminal stent (100) and the at least one stent distal to the luminal stent, and the radial stiffness of the luminal stent (100) is greater than the radial stiffness of the at least one distal stent; and
wherein at least a portion of the radially-expandable stent components (106) are nested.

2. The luminal stent assembly (104) of claim 1, including a plurality of the distal stent components.

3. The luminal stent assembly (104) of claim 2, wherein at least a portion of the distal stent components are nested.

4. The luminal stent assembly (104) of claim 3, wherein the radial stiffness of the at least one distal stent is less than that of the luminal stent (100).

5. The luminal stent assembly (104) of claim 4, wherein the bridges of the luminal stent link at least one of the proximal apices of one of the radially-expandable stent components (106) to a most proximate proximal apex of at least one of the radially-expandable stent component proximal and the radially-expandable stent component distal to the radially-expandable stent component.

6. The luminal stent assembly (104) of claim 5, wherein the bridges of the luminal stent link at least one of the distal apices of one of the radially-expandable stent components (106) to a most proximate distal apex of at least one of the radially-expandable stent component proximal to the radially-expandable stent component and the radially-expandable stent component distal to the radially-expandable stent component.

7. The luminal stent assembly (104) of claim 2, wherein the bridges of the luminal stent link at least one of the distal apices of one of the radially-expandable stent components (106) to a most proximate distal apex of at least one of the radially-expandable stent component proximal to the radially-expandable stent component and the radially-expandable stent component distal to the radially-expandable stent component.

## Patentansprüche

1. Luminale Stentanordnung (104), umfassend:
a) einen luminalen Stent (100), umfassend
i) eine Vielzahl radial expandierbarer Stentkomponenten (106), wobei jede radial expandierbare Stentkomponente ein proximales und ein distales Ende aufweist und mindestens eine der Stentkomponenten Streben umfasst, wobei die Streben gegenüberliegende Enden aufweisen und an den jeweiligen gegenüberliegenden Enden miteinander verbunden sind, wodurch proximale und distale Scheitelpunkte gebildet werden und die radial expandierbaren Stentkomponenten (106) in relativer proximaler und distaler Beziehung zueinander stehen, und
ii) eine Vielzahl von Brücken (28), die unmittelbar proximale und distale radial expandierbare Stentkomponenten (106) miteinander verbinden und dadurch den luminalen Stent (100) bilden und ein kontinuierliches Lumen, ein proximales Ende und ein distales Ende des luminalen Stents (100) definieren;
b) mindestens eine luminale Transplantatkomponente (96) und/oder eine Polymerbeschichtung (154) am luminalen Stent (100); und
c) mindestens einen Stent (102), der distal zum luminalen Stent (100) angeordnet ist und mit dem luminalen Stent (100) durch mindestens die luminale Transplantatkomponente (96) und die Polymerbeschichtung (154) verbunden ist, um dadurch die luminale Stentanordnung (104) zu bilden, wobei die luminale Stentanordnung (104) ein proximales Ende am luminalen Stent (100) und ein distales Ende am Stent distal zum luminalen Stent (100) aufweist,
c) mindestens einen Stent (102), der distal zum luminalen Stent (100) angeordnet ist und mit dem luminalen Stent (100) durch mindestens die luminale Transplantatkomponente (96) und die Polymerbeschichtung (154) verbunden ist, um dadurch die luminale Stentanordnung (104) zu bilden, wobei die luminale Stentanordnung (104) ein proximales Ende am luminalen Stent (100) und ein distales Ende am Stent distal zum luminalen Stent (100) aufweist,
wobei zumindest ein Teil der radial expandierbaren Stentkomponenten (106) ineinander verschachtelt ist.

2. Luminale Stentanordnung (104) nach Anspruch 1, die eine Vielzahl der distalen Stentkomponenten enthält.

3. Luminale Stentanordnung (104) nach Anspruch 2, wobei zumindest ein Teil der distalen Stentkomponenten ineinander verschachtelt ist.

4. Luminale Stentanordnung (104) nach Anspruch 3, wobei die radiale Steifigkeit des mindestens einen distalen Stents geringer ist als die des luminalen Stents (100).

5. Luminale Stentanordnung (104) nach Anspruch 4, wobei die Brücken des luminalen Stents mindestens einen der proximalen Scheitelpunkte einer der radial expandierbaren Stentkomponenten (106) mit einem nächstgelegenen proximalen Scheitelpunkt mindestens einer der radial expandierbaren Stentkomponente proximal und der radial expandierbaren Stentkomponente distal zur radial expandierbaren Stentkomponente verbinden.

6. Luminale Stentanordnung (104) nach Anspruch 5, wobei die Brücken des luminalen Stents mindestens einen der distalen Scheitelpunkte einer der radial expandierbaren Stentkomponenten (106) mit einem nächstgelegenen distalen Scheitelpunkt mindestens einer der radial expandierbaren Stentkomponente proximal zur radial expandierbaren Stentkomponente und der radial expandierbaren Stentkomponente distal zur radial expandierbaren Stentkomponente verbinden.

7. Luminale Stentanordnung (104) nach Anspruch 2, wobei die Brücken des luminalen Stents mindestens einen der distalen Scheitelpunkte einer der radial expandierbaren Stentkomponenten (106) mit einem nächstgelegenen distalen Scheitelpunkt mindestens einer der radial expandierbaren Stentkomponente proximal zur radial expandierbaren Stentkomponente und der radial expandierbaren Stentkomponente distal zur radial expandierbaren Stentkomponente verbinden.

## Revendications

1. Ensemble d'endoprothèses luminales (104), comprenant :
a) une endoprothèse luminale (100) comportant,
i) une pluralité de composants d'endoprothèse radialement expansibles (106), chaque composant d'endoprothèse radialement expansible ayant une extrémité proximale et une extrémité distale, au moins l'un des composants d'endoprothèse comportant des entretoises, dans lequel les entretoises comportent des extrémités opposées et sont jointes l'une à l'autre aux extrémités opposées respectives, formant ainsi des sommets proximaux et des sommets distaux, les composants d'endoprothèse radialement expansibles (106) étant en relation proximale et distale relative l'un par rapport à l'autre, et
ii) une pluralité de ponts (28) reliant les composants d'endoprothèse radialement expansibles (106) immédiatement proximaux et distaux les uns aux autres, formant ainsi l'endoprothèse luminale (100) et définissant une lumière continue, une extrémité proximale, et une extrémité distale de l'endoprothèse luminale (100) ;
b) au moins un composant de greffe luminale (96) et un revêtement polymère (154) au niveau de l'endoprothèse luminale (100) ; et
c) au moins une endoprothèse distale (102) à l'endoprothèse luminale (100) et reliée à l'endoprothèse luminale (100) par au moins l'un du composant de greffe luminale (96) et du revêtement polymère (154) pour former ainsi l'ensemble d'endoprothèses luminales (104), l'ensemble d'endoprothèses luminales (104) ayant une extrémité proximale au niveau de l'endoprothèse luminale (100) et une extrémité distale au niveau de l'endoprothèse distale à l'endoprothèse luminale (100), dans lequel la rigidité axiale de l'ensemble d'endoprothèses luminales (104) diminue de l'extrémité proximale à l'extrémité distale de l'ensemble d'endoprothèses luminales (104) en conséquence d'un espace entre l'endoprothèse luminale (100) et l'au moins une endoprothèse distale à l'endoprothèse luminale, et la rigidité radiale de l'endoprothèse luminale (100) est supérieure à la rigidité radiale de l'au moins une endoprothèse distale ; et
dans lequel au moins une partie des composants d'endoprothèse radialement expansibles (106) sont imbriqués.

2. Ensemble d'endoprothèses luminales (104) selon la revendication 1, comportant une pluralité de composants d'endoprothèse distale.

3. Ensemble d'endoprothèses luminales (104) selon la revendication 2, dans lequel au moins une partie des composants d'endoprothèse distale sont imbriqués.

4. Ensemble d'endoprothèses luminales (104) selon la revendication 3, dans lequel la rigidité radiale de l'au moins une endoprothèse distale est inférieure à celle de l'endoprothèse luminale (100).

5. Ensemble d'endoprothèses luminales (104) selon la revendication 4, dans lequel les ponts de l'endoprothèse luminale relient au moins l'un des sommets proximaux de l'un des composants d'endoprothèse radialement expansibles (106) à un sommet proximal le plus proche d'au moins l'un du composant d'endoprothèse radialement expansible proximal et du composant d'endoprothèse radialement expansible distal au composant d'endoprothèse radialement expansible.

6. Ensemble d'endoprothèses luminales (104) selon la revendication 5, dans lequel les ponts de l'endoprothèse luminale relient au moins l'un des sommets distaux de l'un des composants d'endoprothèse radialement expansibles (106) à un sommet distal le plus proche d'au moins l'un du composant d'endoprothèse radialement expansible proximal au composant d'endoprothèse radialement expansible et du composant d'endoprothèse radialement expansible distal au composant d'endoprothèse radialement expansible.

7. Ensemble d'endoprothèses luminales (104) selon la revendication 2, dans lequel les ponts de l'endoprothèse luminale relient au moins l'un des sommets distaux de l'un des composants d'endoprothèse radialement expansibles (106) à un sommet distal le plus proche d'au moins l'un du composant d'endoprothèse radialement expansible proximal au composant d'endoprothèse radialement expansible et du composant d'endoprothèse radialement expansible distal au composant d'endoprothèse radialement expansible.
